# EUROPEAN PATENT SPECIFICATION

(11) **EP 2 331 955 B1**
(45) Date of publication and mention of the grant of the patent: **30.10.2013**
(21) Application number: 09807801.7
(22) Date of filing: 18.08.2009
(51) Int. Cl.: G01N 33/48, G01N 24/08, G01N 33/483, G01R 33/465, G01N 33/68

(54) **Diagnosis of an asthma disease state**
Diagnose eines Asthma-Krankheitszustandes
Diagnostic d'un état d'asthme

(30) Priority: 18.08.2008 US 136198 P; 15.10.2008 CA 2641131
(43) Date of publication of application: 15.06.2011
(73) Proprietor: The Governors of the University of Alberta, Edmonton, Alberta T5J 4P6 (CA)
(72) Inventor: ADAMKO, Darryl J., Edmonton Alberta T6G 2E1 (CA); SAUDE, Erik, Edmonton, Alberta T6G 2E1 (CA); ROWE, Brian, Edmonton Alberta T6G 2E1 (CA); SYKES, Brian, Edmonton Alberta T6G 2E1 (CA); MOQBEL, Redwan, Winnipeg, Manitoba R3E 0W3 (CA)
(74) Representative: Nederlandsch Octrooibureau
(86) International application number: PCT/CA2009/001314
(87) International publication number: WO 2010/020058

(56) References cited:
- WO-A1-2004/038444
- US-A1- 2005 130 321
- LINDON J C ET AL: "METABONOMICS TECHNOLOGIES AND THEIR APPLICATION IN PHYSIOLOGICAL MONITORING, DRUG SAFETY ASSESSMENT AND DISEASE DIAGNOSIS", BIOMARKERS, TAYLOR AND FRANCIS, LONDON, GB, vol. 9, no. 1, 1 January 2004 (2004-01-01) , pages 1-31, XP009068574, ISSN: 1354-750X, DOI: 10.1080/13547500410001668379
- SAUDE E J ET AL: "NMR-Based Metabolomic Investigation of Asthma and Therapeutic Response", 9TH FRONTIERS OF NMR IN MOLECULAR BIOLOGY KEYSTONE SYMPOSIUM, BANFF, AB, JAN.29-FEB.4, 2005, XX, CA , 29 January 2005 (2005-01-29), page 1, XP008145371, Retrieved from the Internet: URL:www.metabolomics.ca/News/posters/Keyst one.pdf [retrieved on 2012-02-15]
- GILLIES R J ET AL: "NMR Studies of Body Fluids and Tissue Extracts", NMR IN PHYSIOLOGY AND BIOMEDICINE, XX, XX, 1 January 1994 (1994-01-01), pages 221-236, XP002176309,
- SAUDE ERIK J ET AL: "Metabolomic biomarkers in a model of asthma exacerbation: urine nuclear magnetic resonance.", AMERICAN JOURNAL OF RESPIRATORY AND CRITICAL CARE MEDICINE 1 JAN 2009 LNKD- PUBMED:18931331, vol. 179, no. 1, 1 January 2009 (2009-01-01), pages 25-34, XP002669625, ISSN: 1535-4970
- SHOCKOR, J.P. ET AL.: 'Metabonomic applications in toxicity screening and disease diagnosis' CURR. TOP. MED. CHEM. vol. 2, no. 1, 2002, pages 35 - 51, XP008145375
- CARRARO, S. ET AL.: 'Metabolomics applied to exhaled breath condensate in childhood asthma' AM. J. RESPIR. CRIT. CARE MED. vol. 175, 2007, pages 986 - 990
- 'the 9th Frontiers ofNMR in Molecular Biology Keystone Symposium, Banff, AB, Jan.29-Feb.4, 2005', article SAUDE, E.J. ET AL.: 'NMR-based metabolomic investigation of asthma and therapeutic response', XP008145371
- 'the Second Annual Metabolomics Symposium: Exploring the Human Metabolome poster session, Timms Centre, University of Alberta, Oct.12, 2006', article SAUDE, E. J. ET AL.: '`Urinary biomarkers of asthma inflammation in children: the use of NMR spectroscopy', XP008145378

## Description

### FIELD OF THE INVENTION

The invention relates to a method for diagnosing an asthma disease state in a subject.

### BACKGROUND OF THE INVENTION

While diseases of the respiratory system are not responsible for a high mortality rate, they remain among the leading diseases for their impact on society. According to the 1998/99 National Population Health Survey, there were around 2,474,400 Canadians diagnosed with asthma and 498,900 with COPD. These illnesses resulted in 454 and 9,398 deaths, respectively. COPD is the 5th leading cause of death in Canada, and the only one that is increasing in prevalence. The prevalence of asthma is increasing worldwide, and it is the most common chronic disease of childhood. These conditions place a significant burden on the healthcare system, accounting for over $4 billion annually in direct and indirect costs. Community acquired pneumonia (CAP) affects nearly 400,000 Canadians annually and causes death in 8% of hospitalized patients. CAP is estimated to cost the Canadian healthcare system $1 billion per year. The mortality rate associated with pneumonia has increased in the last 20 years despite advances in the treatment of infectious diseases. Bronchiolitis tends to afflict the very young and the very old in society. It is the most common disease requiring hospitalization in paediatrics, with hospitalization rates climbing by 45-55% between 1987 and 1997. As a result, bronchiolitis is one of the leading medical expenses in Canada at $23 million per year.

A feature common to most diseases of the respiratory system is some form of lung inflammation. Lung inflammation consists of specific inflammatory cells and by products generated by cellular activity. Thus, specific lung diseases are often diagnosed not only by their clinical presentation, but also by the type of inflammation measured. Inflammatory cells release enzymes and other proteins in the airway, which can be measured and are specific to the cell type (i.e. mast cell tryptase or eosinophil cationic protein). The treatments for each disease are designed to address this inflammation (i.e. corticosteroids vs. antibiotics). For example, patients with asthma often have sputum samples positive for cells called eosinophils, and those with COPD or pneumonia present with increased sputum neutrophils¹. While babies do not produce sputum, samples from bronchoscopy show neutrophils and eosinophils during asthma exacerbation².

A good example of a lung disease where the link between inflammation and treatment is established is asthma. Asthma is a heterogeneous syndrome with many clinical classifications based on patient symptoms, lung function and response to therapy. The symptoms and the objective measurements of lung function, which clinicians use to guide therapy are largely the result of allergic airway inflammation (i.e eosinophils and mast cells)³. Thus, international guidelines suggest that the desired management goal is to adjust therapy to control inflammation⁴⁵.

Unfortunately for clinicians, detecting this inflammation in individual patients is often difficult. Instead, clinicians rely on physiological (i.e. spirometry, peak flow, airway hyperreactivity (AHR)) or functional measurements (i.e. symptoms, or quality of life) to assess response to therapy. These tests, while useful, appear to be somewhat insensitive to changes in inflammatory status that later become clinically relevant⁶⁷. For example, Green et al. used objective measurements of airway inflammation, induced sputum and/or exhaled nitric oxide (eNO), to guide therapy and demonstrated superior clinical outcomes in their asthma patients⁷. Compared to the traditional measures above, measuring airway inflammation directly detected exacerbations of asthma before lung function or symptom measures changed. Thus, patients had medications adjusted up or down sooner than the traditional treatment group. Overall the amount of corticosteroids used was lower in the group using inflammation measurements.

While accurate airway inflammation measurements from bronchoscopy are possible, it is invasive and unavailable in the daily clinical setting. Thus, research has focused on non-invasive measures of inflammation such as induced sputum, eNO, and various inflammatory markers in body fluids. While experience with sputum has shown valuable results, significant barriers remain to its use clinically, including limited availability in most centers and the inability of young children and even many adults to expectorate. Exhaled NO overcomes some of these barriers; however, while eNO shows correlation with asthma inflammation and outcomes experimentally, it requires time, co-operation, age greater than 4 years and, unless very carefully performed, may measure sinus rather than airway values⁸. Ultimately, eNO still lacks the sensitivity and specificity of induced sputum. While other tests for asthma inflammation in blood or urine have been studied, i.e. urine leukotrienes, or eosinophil protein X, they lack the sensitivity required for clinical practice. Overall, a simple, non-invasive, readily available and sensible test for patients with airway inflammation is currently not widely available.

Several techniques are known that may be used for the identification of biomarkers, i.e. metabolites in biofluids such as urine. Many known techniques involve extensive sample preparation which may destroy the original sample. Furthermore, in some of these techniques the detection of the biomarkers can be limited.

Other non-invasive techniques have also been used which involve the use of a single biomarker in the identification of a disease state. Such techniques can be limiting in the detection of disease states that are not dependent on a single factor, such as diseases of the respiratory system.

The article Carraro S. et al., "Metabolomics Applied to Exhaled Breath Condensate in Childhood Asthma", Am. J. Respir. Crit. Care Med. 2007, pp. 986-990 [see reference 78] describes a method of diagnosing asthma in a subject, the method comprising obtaining nuclear magnetic resonance data on an obtained biological sample from the subject in the form of an x,y trace, performing a statistical analysis on the nuclear magnetic resonance data, determining a subject profile for the biological sample based on the statistical analysis and comparing the subject profile to a predetermined profile to provide a diagnosis of the disease state.

The poster Saude E.J. et al., "Urinary Biomarkers of Asthma Inflammation in Children: The use of NMR Spectroscopy", presented at the Second Annual Metabolomics Symposium, University of Alberta, Oct. 12, 2006 refers to the detection of asthma via NMR of urinary biomarkers. It is suggested to continue the work by means of multivariate analysis to determine the ability to diagnose and provide a prognosis through NMR analysis of asthma urine.

### SUMMARY OF THE INVENTION

There is provided a method of diagnosing an asthma disease state in a subject. The method is set forth in claim 1, the dependent claims defining preferred embodiments.

### BRIEF DESCRIPTION OF THE DRAWINGS

The methods disclosed herein will now be described in further detail with reference to the following figures:

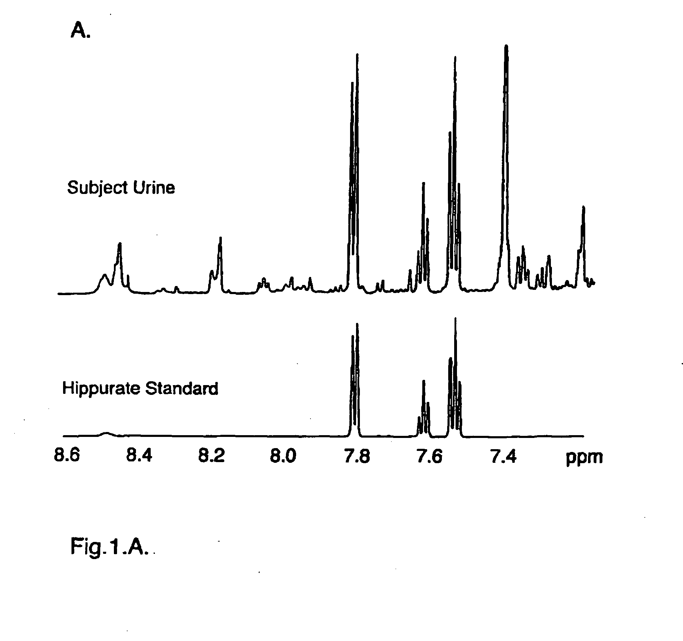

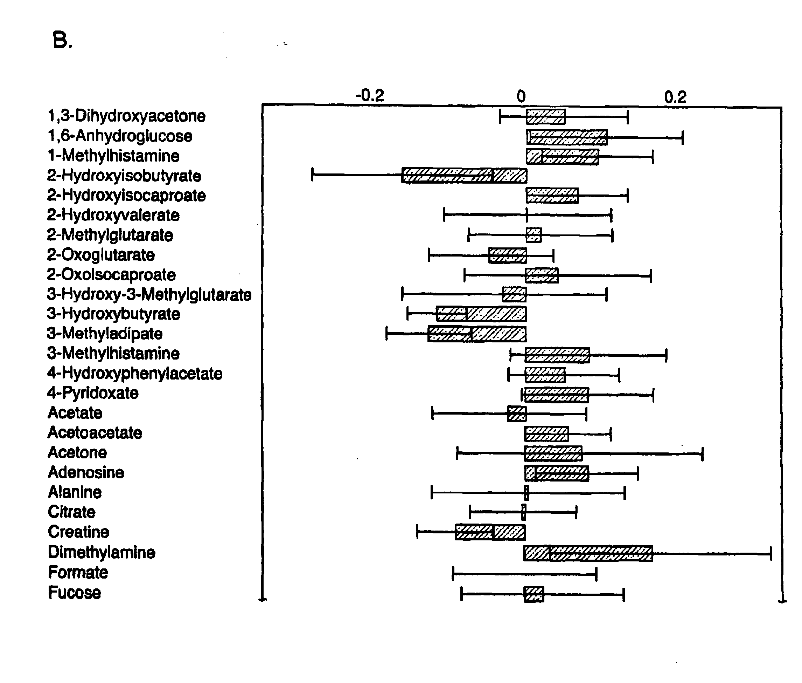

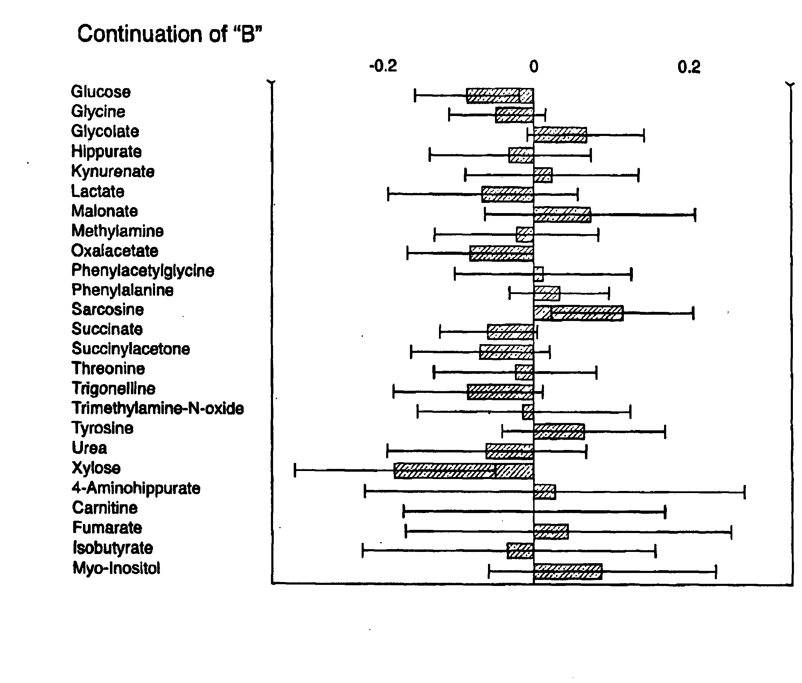

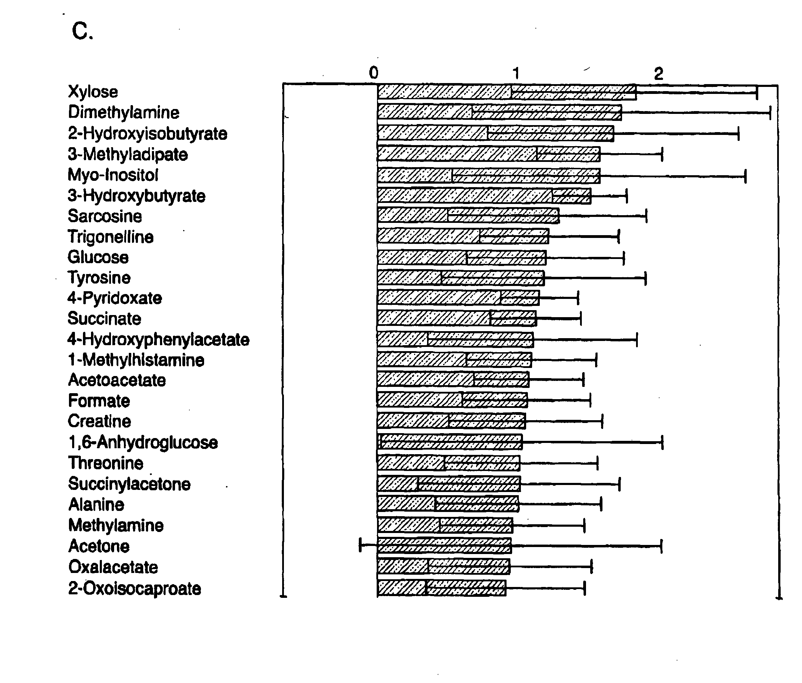

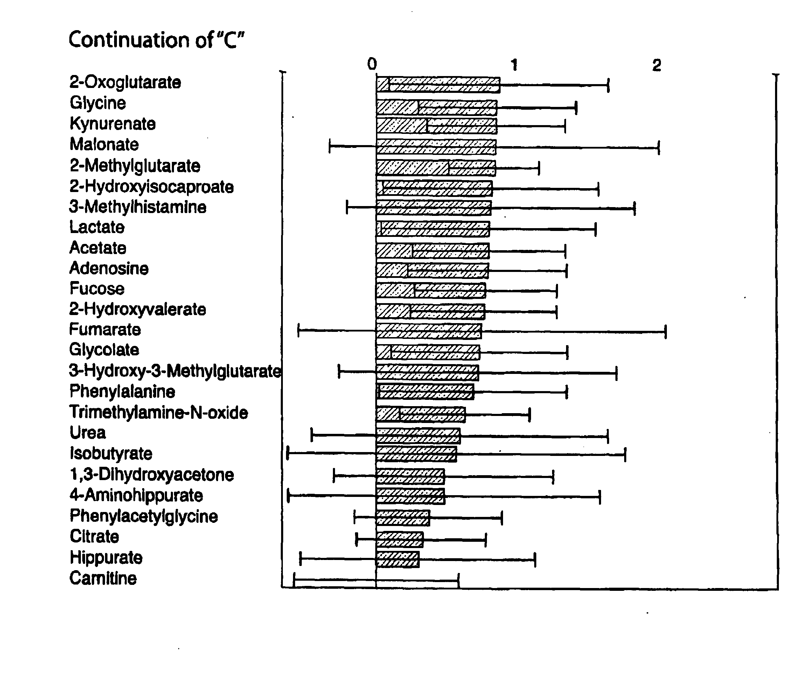

Figure 1A is 600 MHz 1D ¹H-NMR spectrum of a guinea pig urine sample above a referenced trace of resonant signatures for hippurate;

Figure 1B is a Coefficient of Variation plot between challenged vs. sensitized guinea pigs for all metabolites;

Figure 1C is a Variable of Importance plot for challenged vs. sensitized guinea pigs;

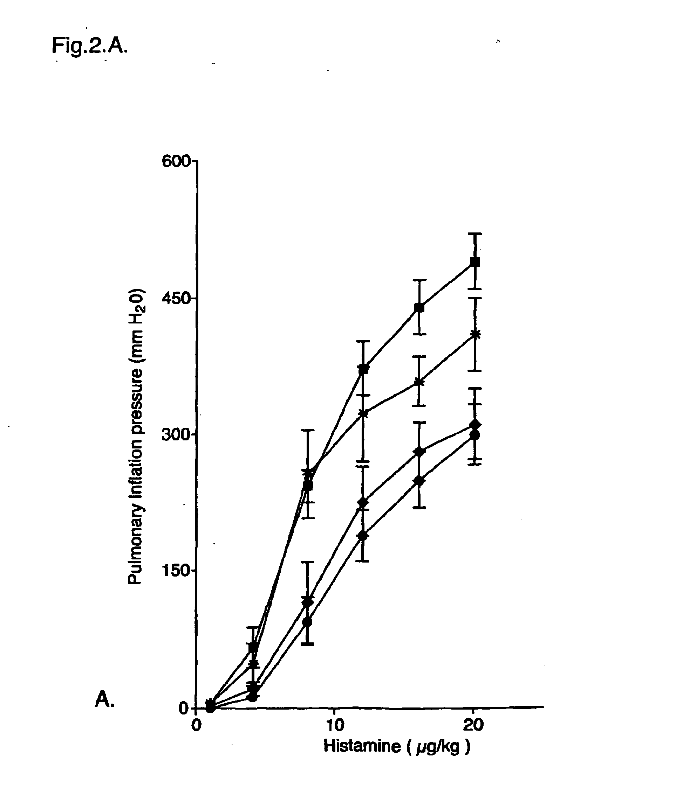

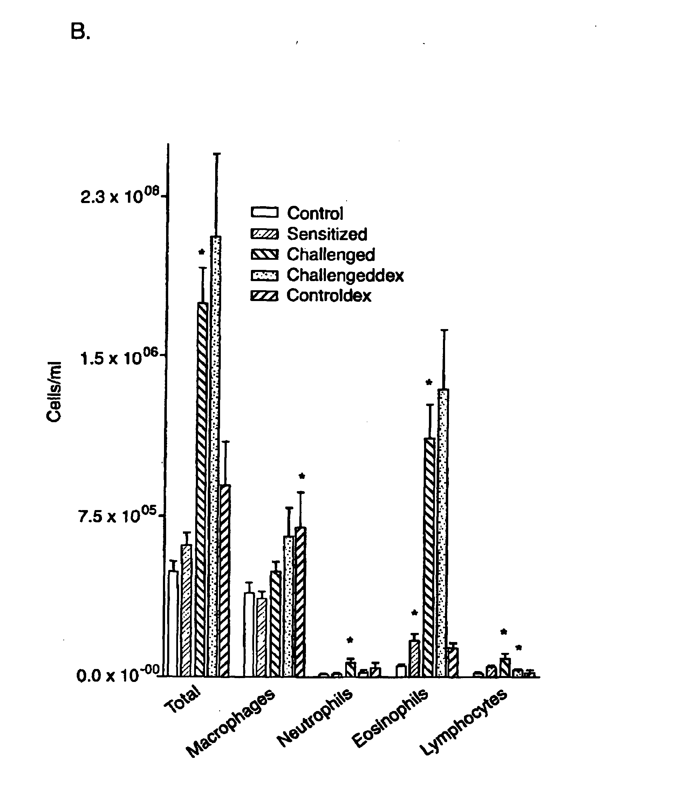

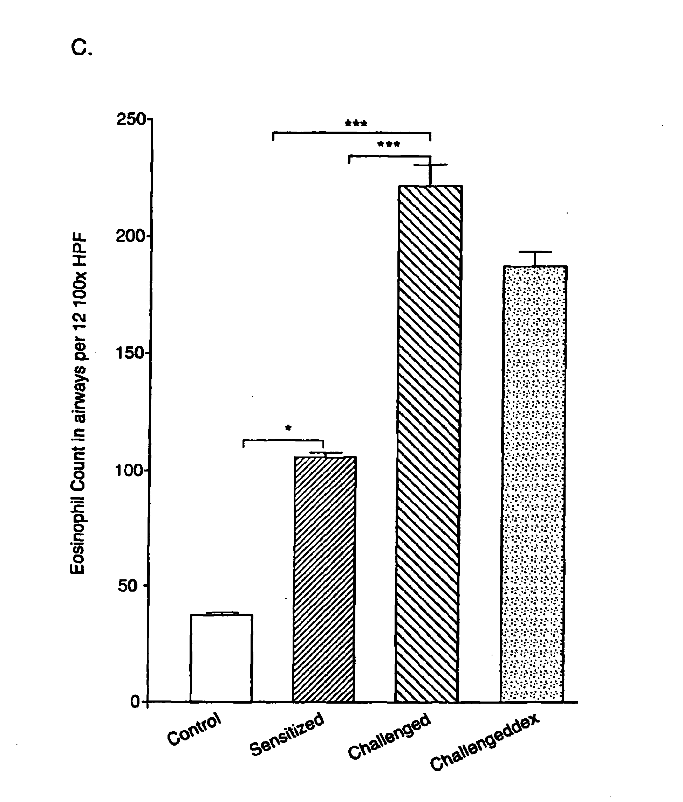

Figure 2A is a graph showing histamine (1-20µg/kg i.v.) induced dose-dependent bronchoconstriction, measured by increasing Ppi in guinea pigs;

Figure 2B is a graph showing total cells in the lung lavage of challenged guinea pigs;

Figure 2C is a graph showing eosinophil count in the airways for different guinea pig groups;

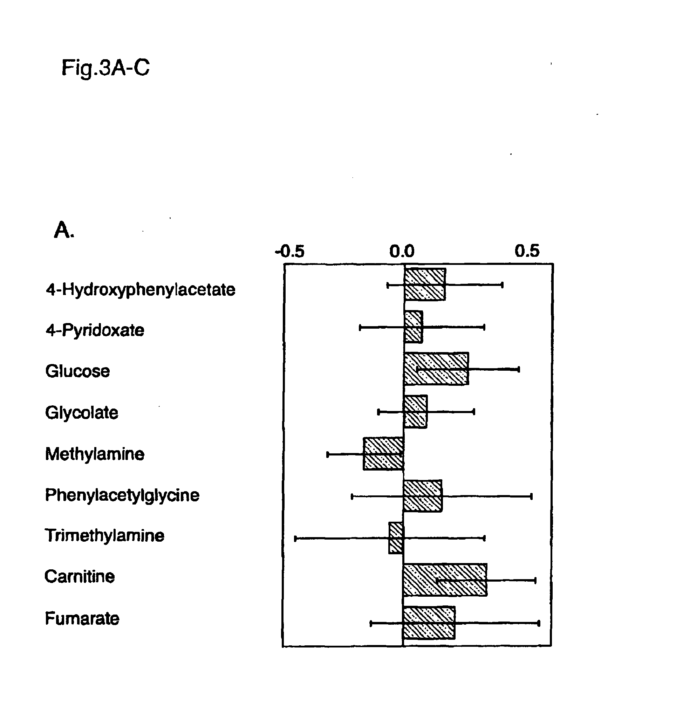

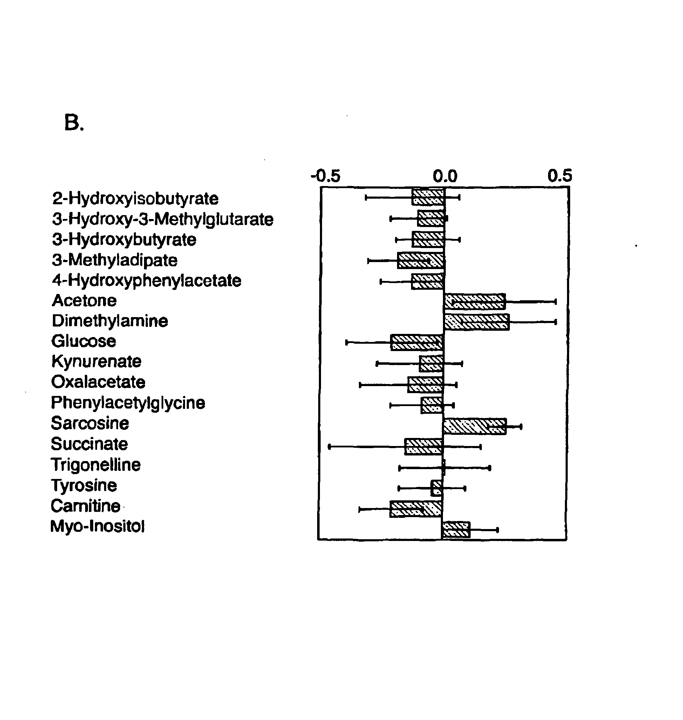

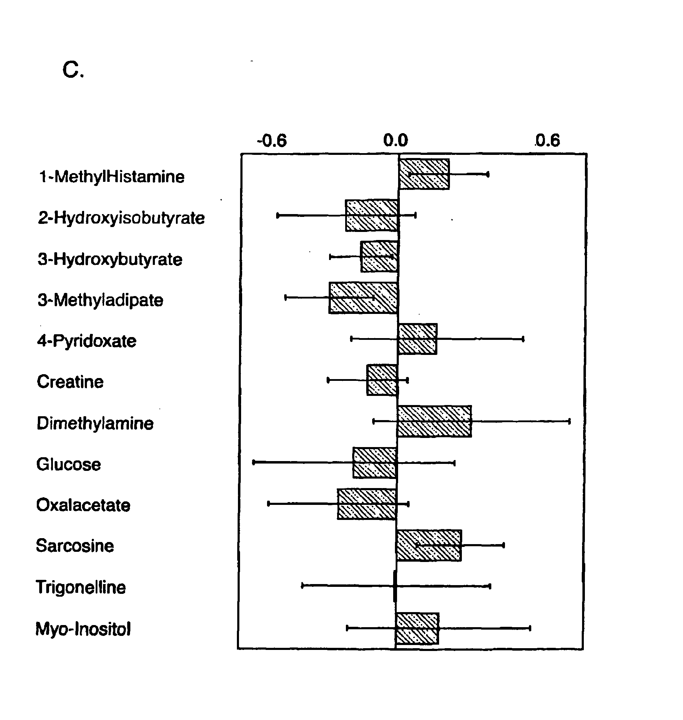

Figures 3A-C are Coefficient of Variation plots for NMR-derived urine metabolite concentrations used by PLS-DA in the final modeling of separation between guinea pig groups: control vs. sensitized guinea pigs (A), control vs. challenged (B), and sensitized vs. challenged (C). (bars represent 95% confidence intervals);

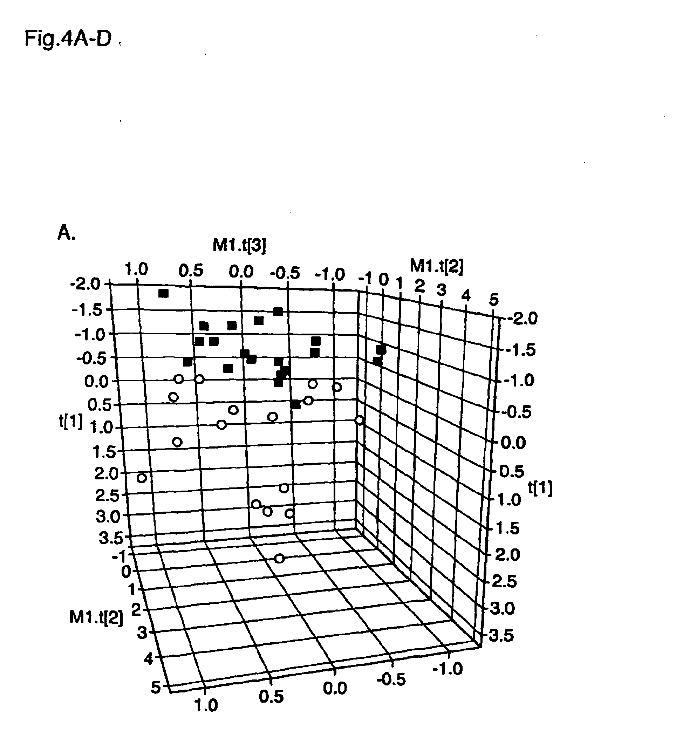

Figures 4A-D shows three-dimensional plots illustrating PLS-DA separation of guinea pig groups based on NMR-derived urine metabolite concentrations; (A) control (circles) vs. sensitized (squares) (R²= 0.53 Q²=0.29); (B) control vs. challenged (triangles) (R²= 0.74 Q²=0.59); (C) sensitized vs. challenged (R²=0.63 Q²=0.50); and (D) 3-way analysis of all animal groups (R²=0.54 Q²=0.25).

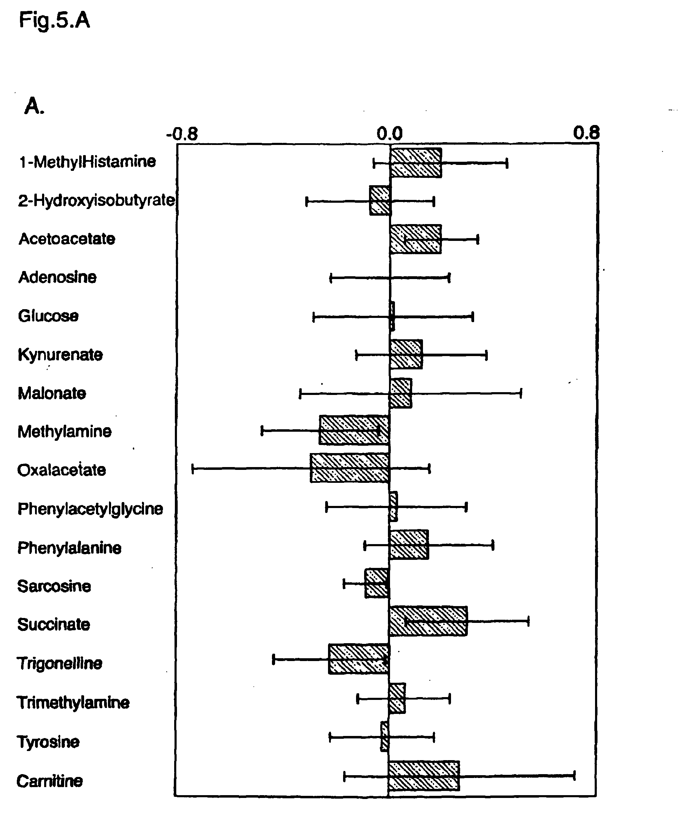

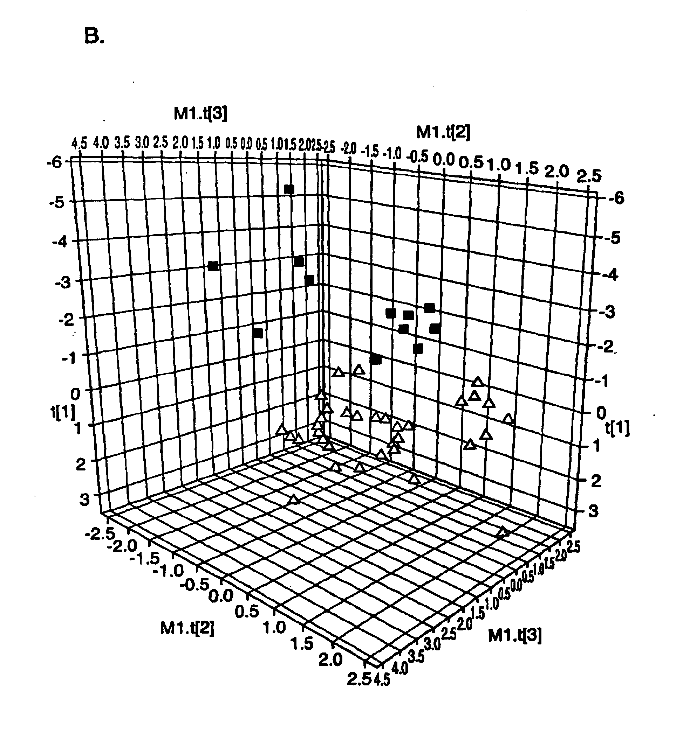

Figure 5A is a Coefficient of Variation plots of NMR-derived urine metabolite concentrations used to separate challenged and challenged plus dexamethasone treated guinea pig groups (bars represent 95% confidence intervals). Figure 5B is a PLS-DA visualization of separation between challenged and challengeddex groups;

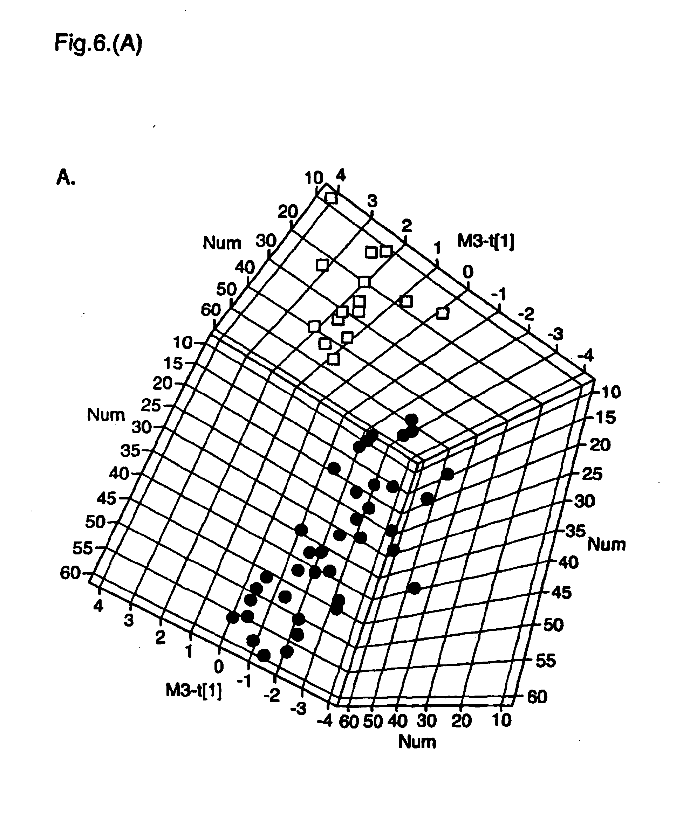

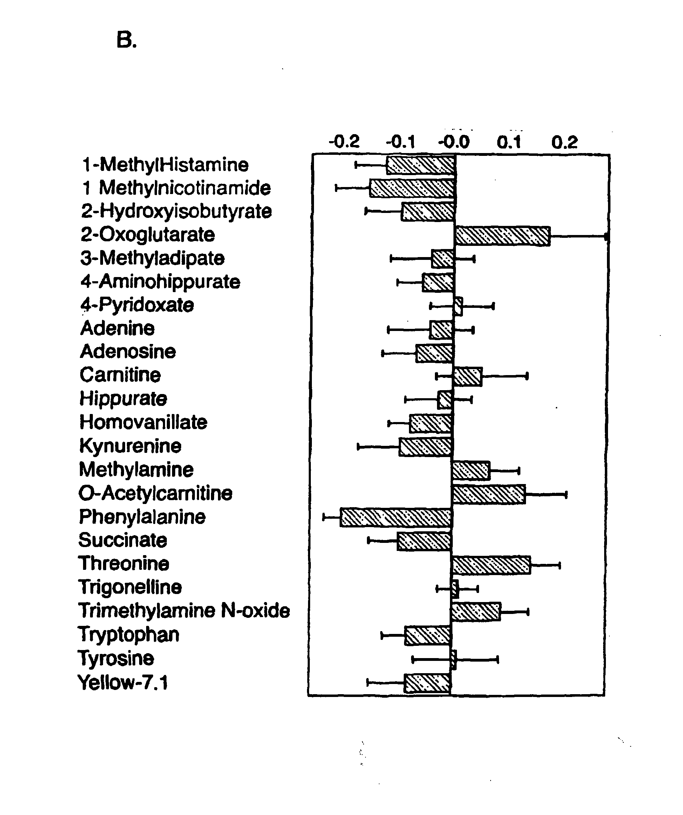

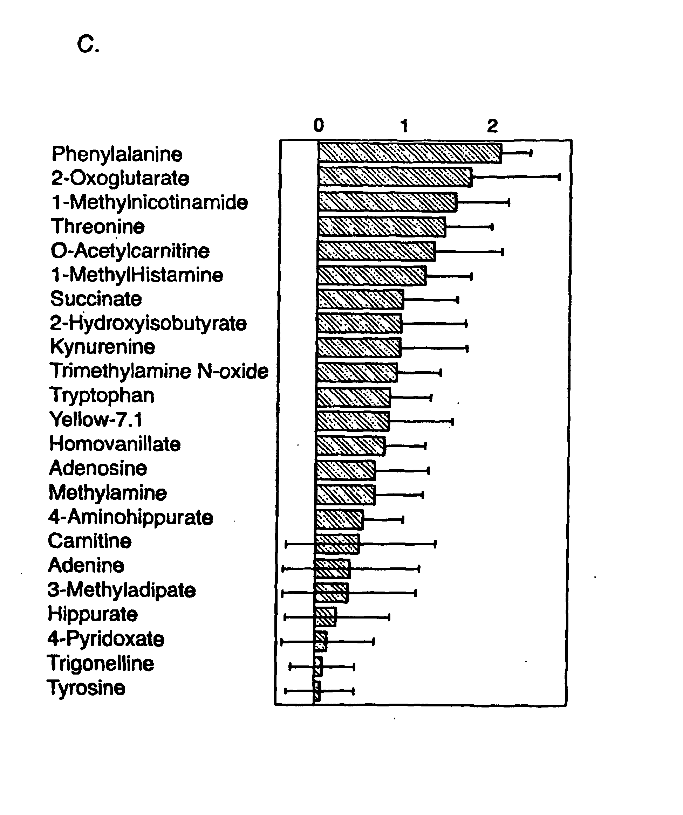

Figures 6. (A) Shown is a three-dimensional plot illustrating PLS-DA separation of healthy control children versus those with asthma in outpatient clinic. The plot is generated from known metabolite concentrations analyzed by PLS-DA as shown as a Coefficients of Variation Plot (B). The metabolites used can be ranked in terms of their importance in the model as seen in the Variables of Importance Plot (C).

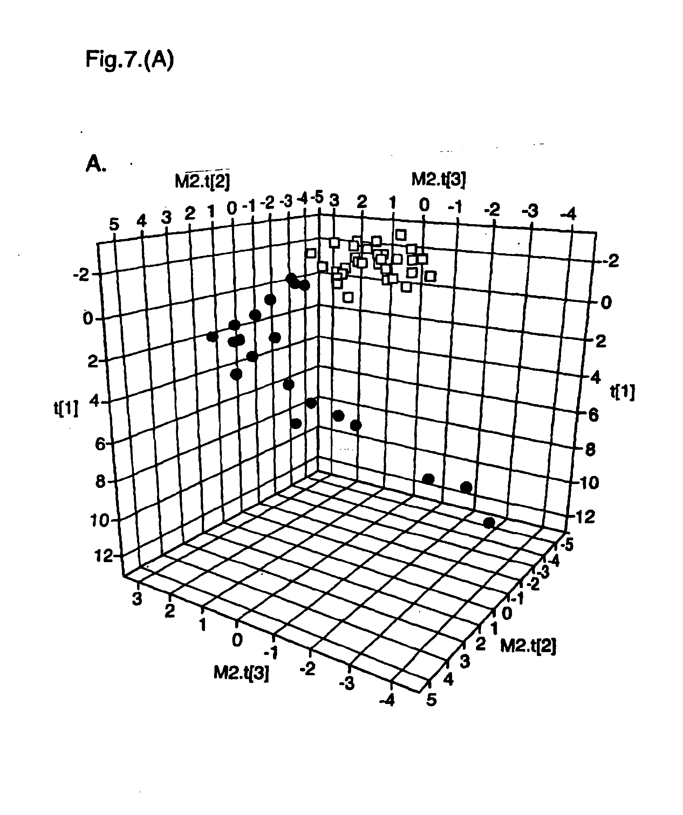

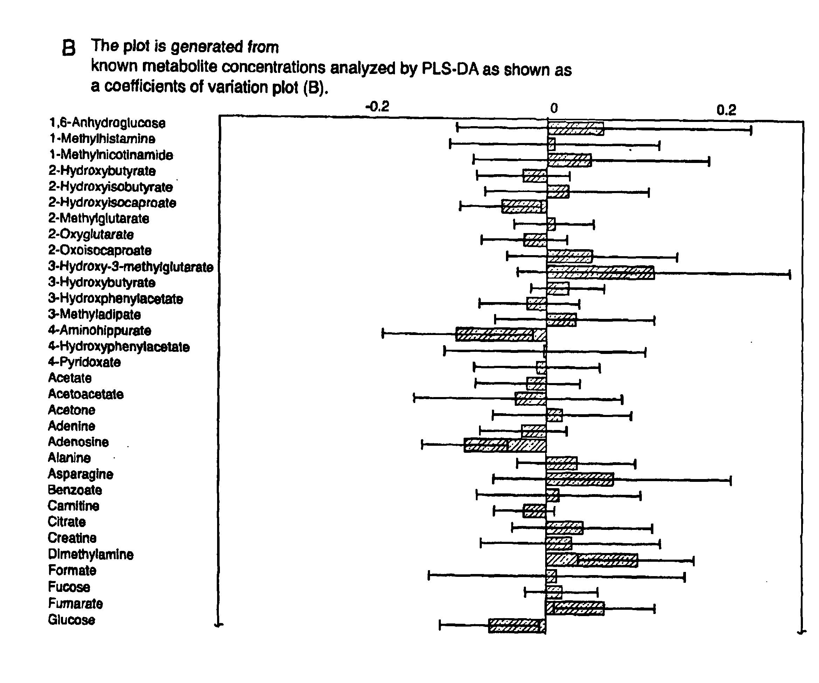

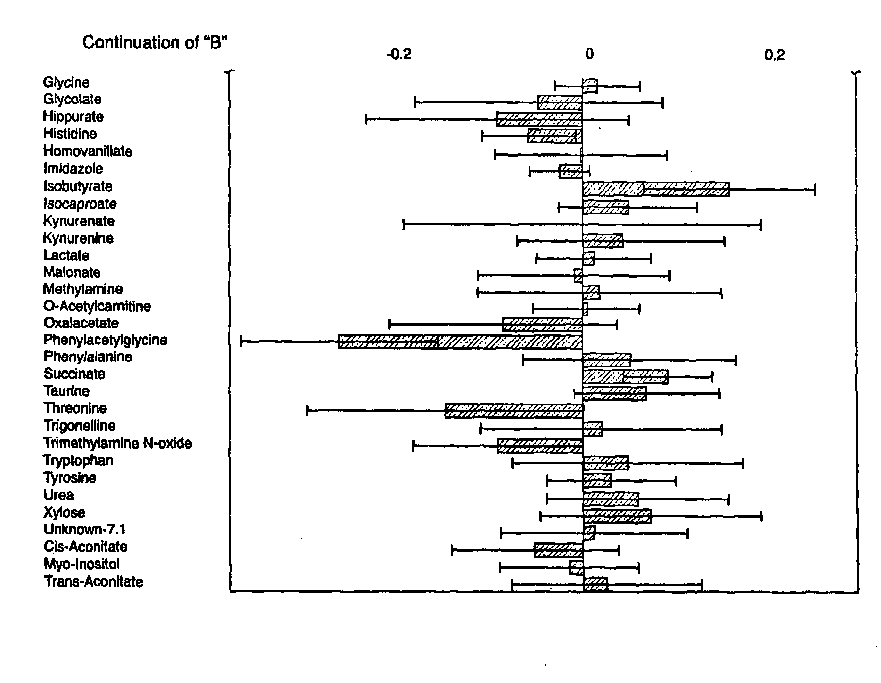

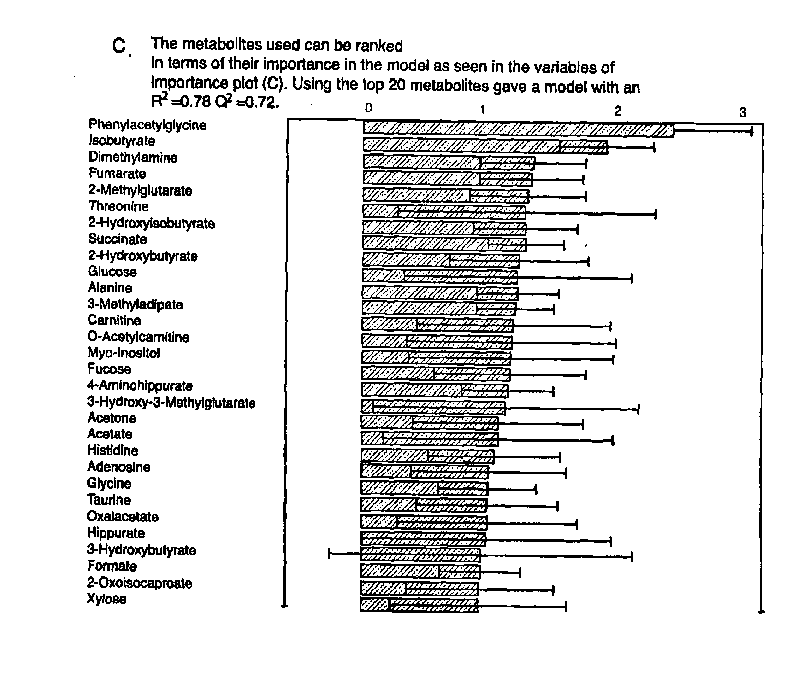

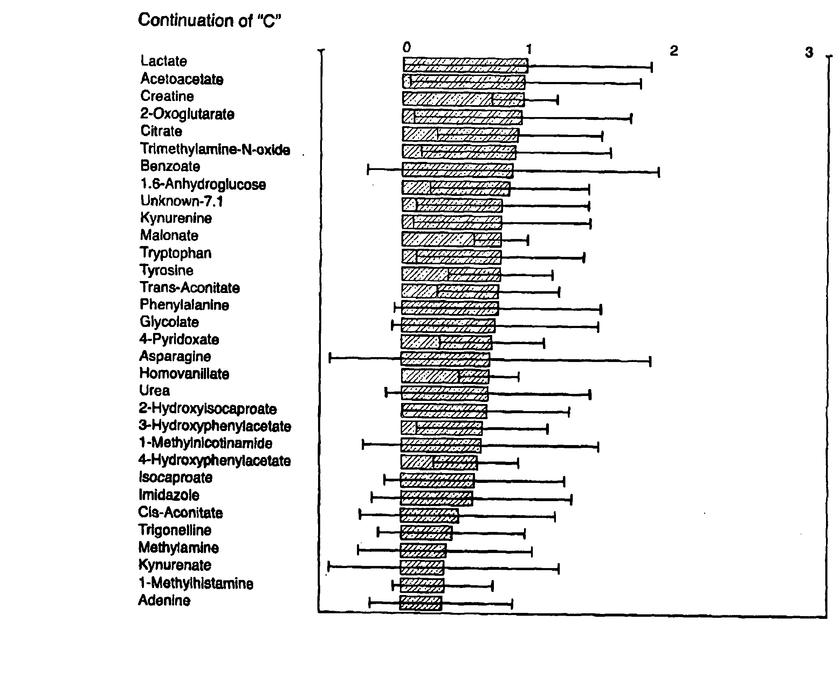

Figures 7. (A) Shown is a three-dimensional plot illustrating PLS-DA separation of healthy control children versus those with asthma in Emergency Department. The plot is generated from known metabolite concentrations analyzed by PLS-DA as shown as a Coefficients of Variation Plot (B). The metabolites used can be ranked in terms of their importance in the model as seen in the Variables of Importance Plot (C).

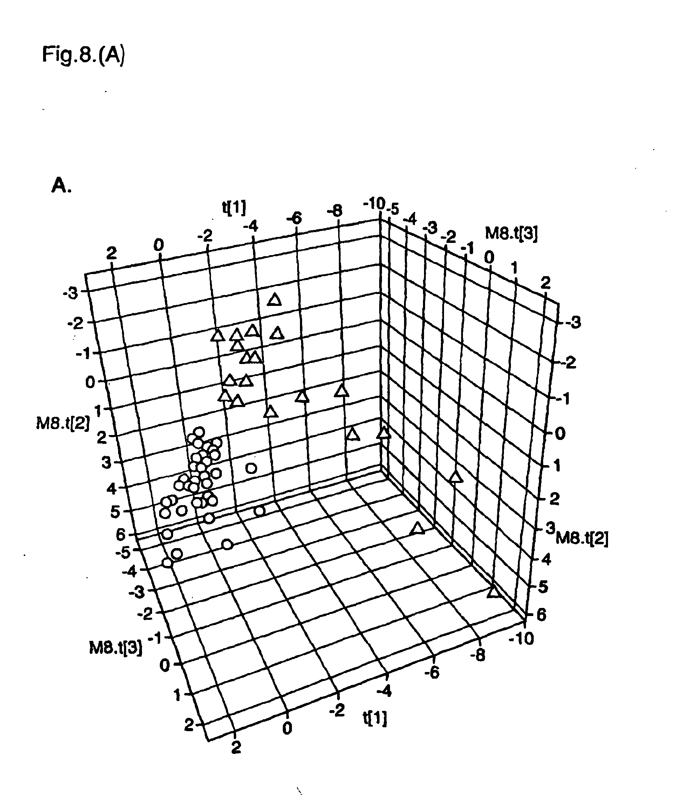

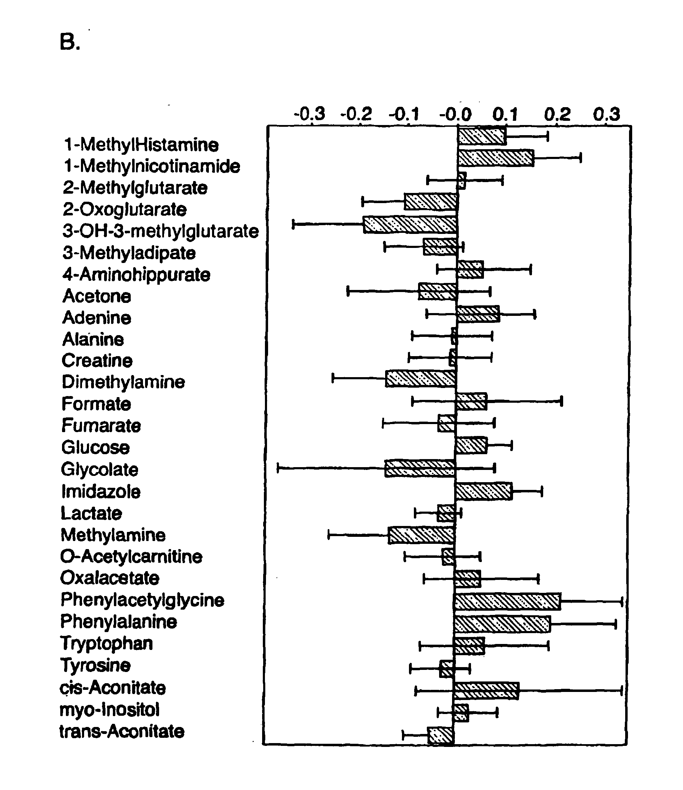

Figures 8. (A) Shown is a three-dimensional plot illustrating PLS-DA separation of children with stable asthma in outpatient clinic versus those with asthma in Emergency Department. The plot is generated from known metabolite concentrations analyzed by PLS-DA as shown as a Coefficients of Variation Plot (B). The metabolites used can be ranked in terms of their importance in the model.

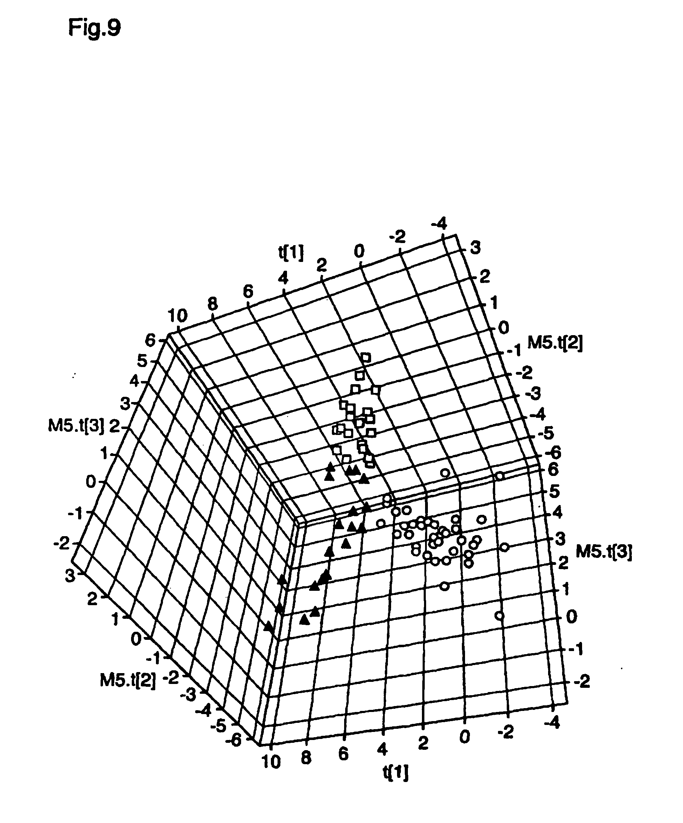

Figures 9. Shown is a three-dimensional plot illustrating PLS-DA separation of all 3 groups of children, healthy (open squares) versus those children with stable asthma in outpatient clinic (open circles) versus those with asthma in Emergency Department (closed circles). The plot is generated from known metabolite concentrations analyzed by PLS-DA. Using the top 30 metabolites gave a model with an R2=0.74, Q2=0.61.

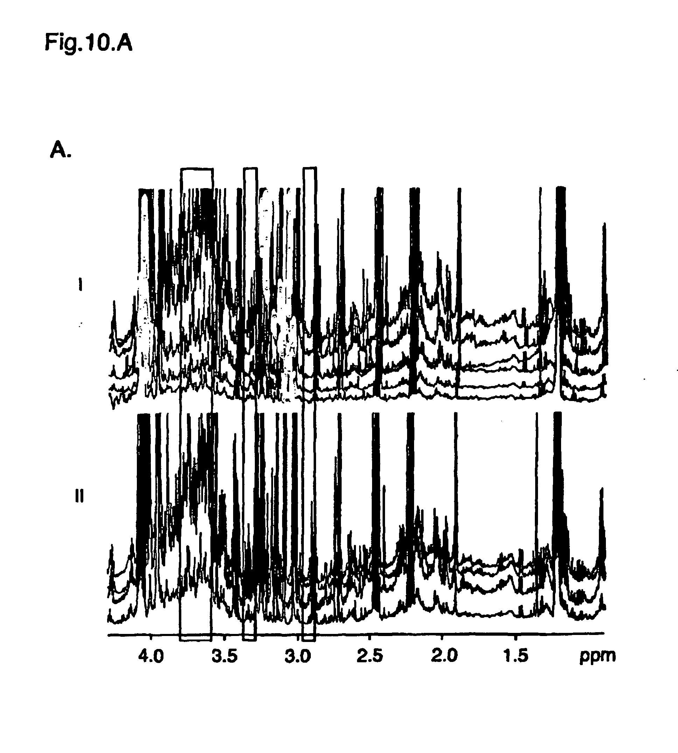

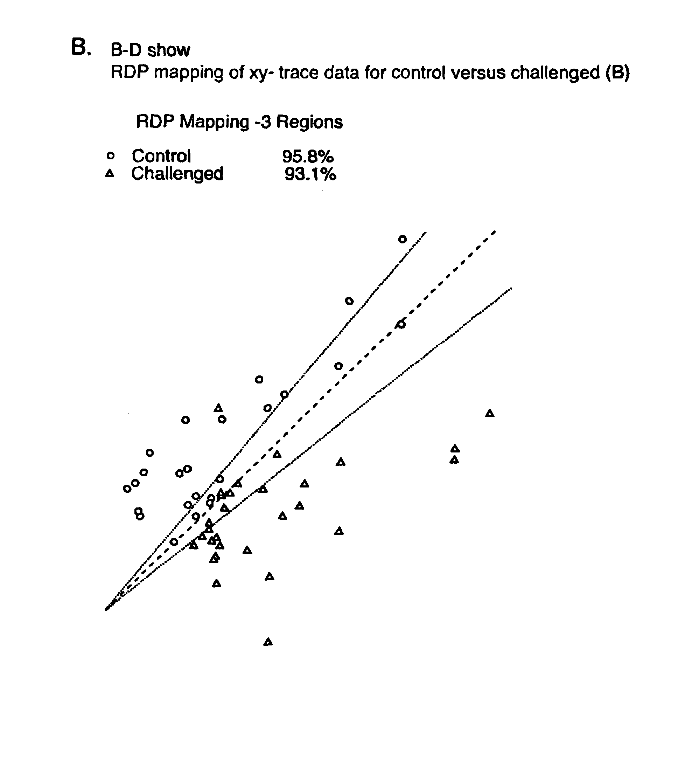

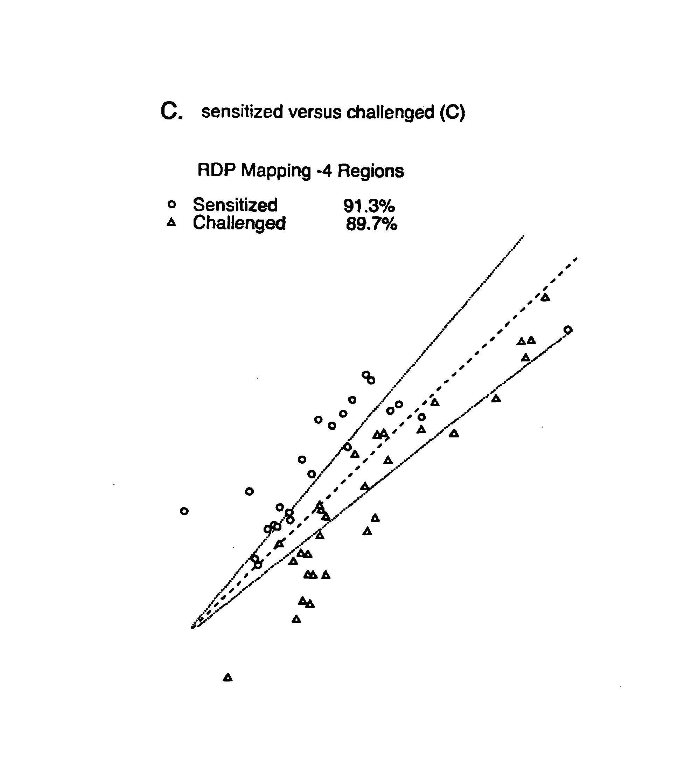

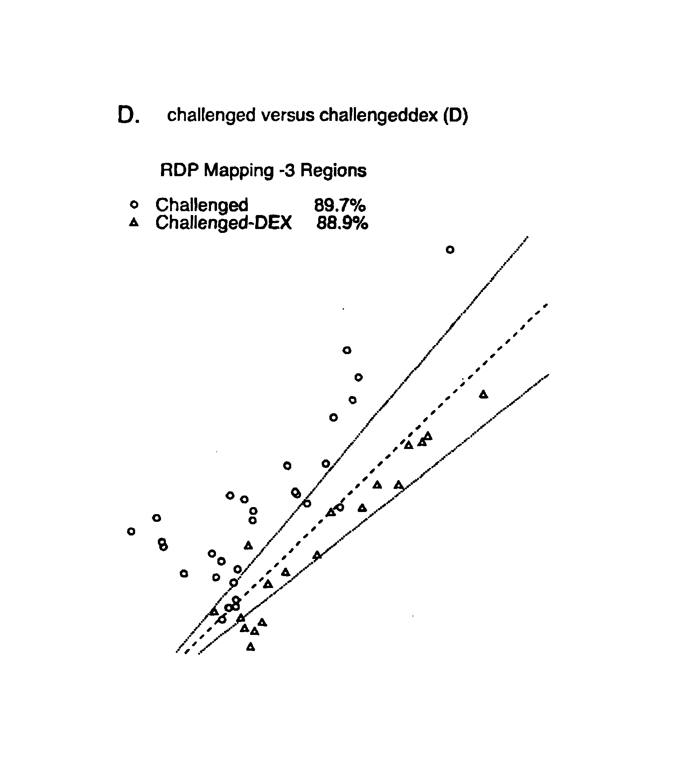

Figure 10A is a plot of 1D ¹H-NMR spectra from some challenged (I) and control (II) guinea pigs, illustrated as a stack of six individual results. Figure 10B-D shows RDP mapping of xy-trace data for guinea pig groups of control versus challenged (B); sensitized versus challenged (C) and challenged versus challenged-dexamethasone (also referred to as "challengeddex") (D).

### DETAILED DESCRIPTION

Asthma is characterized by shortness of breath due to reversible airway obstruction and abnormal airway reactivity to various stimuli. The airway pathology found in patients with asthma is a unique mix of abnormal structural cells^{9, 10} and inflammatory cells³, which are not commonly described in other airway diseases. The severity of asthma and the degree of airway hyperreactivity (AHR) correlates with the presence and magnitude of airway inflammation in the airways¹¹. Thus, asthma management has relied upon the control of inflammation⁵. Improving the ability to accurately monitor airway dysfunction and inflammation through noninvasive means is a key goal in managing asthma therapy.

Metabolomics is the study of metabolic pathways and the measurement of unique biochemical molecules generated in a living system¹². Metabolites are small, non-peptide molecules with molecular weights less than 1 kDa¹³ and are the end products from cellular activity. Detecting changes in metabolite concentrations reveals the range of biochemical effects induced by a disease condition or its therapeutic intervention. ¹H-nuclear magnetic resonance spectroscopy (NMR) allows for the characterization and quantification of these metabolites in biological fluids. The main advantage of using NMR is its ability to provide a rapid and accurate metabolic picture with minimum sample pretreatment^{14, 15}. The advantages of urine include its noninvasive collection and wide availability, its low protein and cellular levels, and its richness in metabolites.

Guinea pigs are a reliable animal model of asthma as their airway physiology is uniquely similar to that of humans^{16, 17}. Guinea pigs that are allergen sensitized and then challenged by aerosolization of the allergen develop airway inflammation, increased work of breathing, a period of hypoxia, and then airway hyperreactivity (that lasts for several days). This is similar to humans with allergies. The use of this animal model of allergic asthma, is herein described to show the effect of airway inflammatory cells on the airways by producing a unique pattern of metabolites in the body, which are excreted in the urine. These urine metabolites may be measured using NMR spectroscopy and used as a biomarker panel to discriminate the subtypes of animals. The relevance of this methodology in human disease is also described. Urine samples from (i) healthy children, and (ii) those from children with asthma have been studied. The populations of asthma patients include those children that are stable in outpatient clinic, and those that are quite ill in the emergency department.

There is provided a method of diagnosing an asthma disease state in a subject.

The methods described herein require that a biological test sample is obtained from a subject. The biological test sample may be selected from the group consisting of blood, blood plasma, blood serum, saliva, pleural fluid, nasal fluid, intracellular fluid, intercellular fluid, lymph fluid, cerebrospinal fluid, bile acid, synovial fluid, pericardial fluid, peritoneal fluid, feces, ocular fluid, tissue, sputum, and urine. In one embodiment, the biological test sample is urine.

From the sample, the concentration of metabolites is determined using nuclear magnetic resonance spectroscopy.

In addition, from the sample, the values from the xy-trace data of the NMR spectra may be obtained.

The final profile for the biological test sample is determined by performing a statistical analysis on the data (i.e. the concentration of certain metabolites or the xy-trace data). The type of statistical analysis that is used is a multivariate statistical analysis, selected from the group of principal component analysis, discriminant analysis, principal component analysis with discriminant analysis, partial least squares, partial least squares with discriminant analysis, canonical correlation, kernel principal component analysis, non-linear principal component analysis, factor analysis, multidimensional scaling and cluster analysis.

The concentration or combination of relevant metabolites for each disease state determines the diagnosis of disease and/or the severity of a known disease. In one embodiment, the profile of the biological test sample (the subject profile) and the predetermined profile are shown as score plots determined from multivariate statistical analysis. For example, Table 1 lists the concentration of relevant metabolites separating the different groups of guinea pigs within an asthma model of allergen sensitization and challenge. Based on the guinea pig work, the same technique was implemented using urine from humans with asthma compared to healthy controls (Table 2). Table 3 lists the relevant metabolites and their concentration which separate children with asthma from those without asthma or those sicker children having an asthma exacerbation. Thus, these metabolites could not only be used to diagnose asthma, but also monitor children with asthma to determine when their urine NMR profile suggests impending asthma attack.

Each one of the terms "concentration" and "concentration value" is a concentration or a numerical value associated with or derived from a concentration, including a numerical value resulting from a statistical analysis of a concentration or a numerical value associated with or derived from a concentration.

The methods will now be described in detail with reference to the accompanying Figures and the examples described below.

### Methods used in animals and humans

### (A) Testing of Animal (Guinea Pig) Subjects

### Characteristics of Animals and Their Preconditioning:

Female Dunkin-Hartley guinea pigs (GP) were used (specific pathogen-free, 180-450 g, Charles River Laboratories, Saint-Constant, Canada) in accordance with standards established by the University of Alberta Health Sciences Animal Policy and Welfare Committee and Guidelines of the Canadian Council on Animal Care. All guinea pigs were allowed to adapt to the new environment for at least one week before physiologic studies. Based on previous work in this asthma exacerbation model¹⁷⁻¹⁹, guinea pigs were divided into five groups: 1) untreated, nonsensitized controls (control, n=18); 2) control GP administered dexamethasone-water soluble (Sigma-Aldrich, Ontario, Canada) at 6.5 µg/kg i.p. daily for three days (controldex, n=4); 3) sensitized to ovalbumin (OVA) alone (Sigma-Aldrich) at 10 mg/kg i.p. on days 1 and 3 and left for 21 days (sensitized, n=18), 4) sensitized GP were challenged after 21 days with aerosolized ovalbumin 0.5% in 0.9% saline for 10-20s (challenged, n=29), and finally 5) some of the challenged animals were treated with dexamethasone at 6.5 µg/kg i.p., 1 hour after challenge and then daily for two consecutive days (challengeddex, n=12). Airway function and inflammation were assessed four days after OVA challenge or 21 days after sensitization alone.

### Assessment of Airway Hyperreactivity (AHR) in animal subjects:

Guinea Pigs were anesthetized with urethane (1.5 g/kg i.p.), tracheostomized, and ventilated after paralysis with succinylcholine chloride (Sigma-Aldrich) (10 µg/kg/min, i.v.). Pulmonary inflation pressure (Ppi) was measured using (Powerlab, AdInstruments, Colorado Springs, USA) as previously described¹⁷. To assess airway reactivity, histamine (Sigma-Aldrich) was administered at 6-minute intervals (1-20 µg/kg i.v.). The resulting bronchoconstriction was recorded as increases in Ppi.

### Assessment of Airway Inflammation in animal subjects:

Animals were sacrificed with an overdose of urethane (3 gm/kg i.v.). Cytosmear for total and differential cell counts (Diff-Quik®, Baxter Healthcare Corp) were performed on lung lavage ¹⁷. The lungs were removed, inflated and fixed in 3.7% formaldehyde for 24 hours. Airway sections were stained with 2% chromotrope 2R to determine the average number of eosinophils per 10 high power field (#/hpf) as previously described²⁰.

### Statistical Analyses of airway reactivity and inflammation in animal subjects:

The bronchial reactivity measurements (histamine responses) were analyzed using two-way analysis of variance (ANOVA) for repeated measures and histological measurements and lung lavage data were analyzed using ANOVA (Statview 4.5; Abacus Concepts, Inc. Berkley, CA). The results are expressed as mean and standard error of the mean (SEM) and standard deviation (SD) respectively. A P-value of 0.05 was considered significant.

### (B) Testing of Human Subjects

### Characteristics of human subjects used in the study:

Children were recruited from the Stollery Children's Hospital asthma clinic and emergency department (ED) (Table 2). Children with outpatient asthma were initially referred to a pediatric pulmonary or allergy subspecialist, and enrolled after at least one clinical visit having met diagnostic criteria for asthma as described in the Canadian Consensus guidelines²¹. Children in the ED with acute asthma were diagnosed based on one or more of the following: a) increasing symptoms (cough, wheeze, shortness-of-breath, chest tightness) requiring assessment and a history of similar episodes; b) clinical or symptomatic response to inhaled bronchodilator therapy; and/or c) had a previous history of physician-diagnosed asthma. Patients had to present primarily for acute asthma (not a simple prescription refill) and were excluded if they had acute pneumonia, needed immediate resuscitation (status asthmaticus), had cognitive impairment, or had a known immunodeficiency. Healthy age and sex-matched controls were recruited from the general pediatric outpatient clinic and the community. Healthy controls were excluded if they had any underlying lung disease (i.e. chronic cough or wheeze, CF, asthma, immunodeficiency, or oral steroid use) proven malignancy, chronic inflammatory/infective disorder, cardiac disease, and neonatal lung disease associated with prematurity. Patients were enrolled into the study after written informed consent was obtained as approved by the University of Alberta Health Research Ethics Board, in accordance with the Declaration of Helsinki and Good Clinical Practice guidelines.

### (C) Methods Used to Analyze Animal and Human Data

### Collection of human and animal urine samples for determination of one-dimensional ¹H-NMR spectra:

Regarding animal urine data: under anaesthesia, but before performing airway inflammatory measurements, urine samples (1.0-2.0 cc) were collected by trans-abdominal cystocentesis. Regarding human urine data: midstream urine samples were collected in standard 50ml specimen containers and promptly placed in a freezer at the outpatient clinic (-20°C). Within 3 hours each the urine sample was moved to the -80°C freezer at NANUC (National High Field NMR Centre), University of Alberta. To determine the NMR spectra, the samples were thawed in a biosafety fume hood and a 630 µl aliquot was removed and placed in a 1.5 ml Eppendorff tube followed by the addition of 70 µl of a reference buffer solution (4.9 mM DSS (disodium-2, 2-dimethyl 2-silaperitane-5-sulphonate) and 100 mM imidazole in D₂O, Sigma-Aldrich). Each sample was brought to a pH of 7.0 +/- 0.1 using HCl and NaOH. An uncentrifuged aliquot of 600 µl was taken and transferred to a standard 5 mm glass NMR tube (Wilmad, NJ). ¹H-NMR spectra were acquired on a 600 MHz Inova spectrometer (Varian Inc, Palo Alto, CA) equipped with a 5 mm triple-resonance (HCN) probe with z-axis gradient coil. One-dimensional ¹H-NMR spectra were collected at 25°C with a tnnoesy pulse sequence (one-dimensional, three pulse NOESY, with a transmitter pre-saturation delay of 900 ms for water suppression during the pre-acquisition delay and 100 ms mixing time) and a spectral width of 7200 Hz. The time-domain data points were 64k complex points, acquisition time was 4s, 90° pulse was 6.8µs, repetition time was 5s, with four steady state scans, and 32 acquired scans per FID (Free Induction Decay). The data were apodized with an exponential window function corresponding to a line broadening of 0.5 Hz, zero-filled to 128k complex points, and Fourier transformed²²_{.}

### Quantification of Known Metabolites:

Quantification of 50-70 easily identifiable metabolites involved in various relevant metabolic pathways was performed using Chenomx NMR Suite Professional software package Version 3.1 (Chenomx Inc., Edmonton, AB)²³. The software contains a database of known metabolites with their referenced spectral resonant frequencies or signatures. The software allows matching of these known resonant frequencies with the observed resonant frequencies of the collected spectra, enabling the qualitative and quantitative analysis of metabolites in urine NMR spectra (Figure 1A). The methyl groups from DSS produce a resonant singlet, which served as internal standard for spectral chemical shifts (set to 0 ppm) and for quantification. The internal DSS signal was also utilized as the concentration reference (0.49 mM). To correct for dilution, metabolite concentrations were referenced against urinary creatinine²⁴. This method is capable of providing metabolite concentration accuracies in excess of 90%^{23, 25}. While there is daily variation in the excretion of metabolites, no variation was identified that was attributed specifically to diet in humans^{24, 26}.

### Separation of Groups Based on Known Metabolites:

To separate groups of animals or patients based on urine metabolite concentrations, the values of the 50-70 metabolite concentrations for each animal/child were log transformed and imported to SIMCA software (SIMCA-P 11, Umetrics, USA). As seen in the human data²⁴, the concentrations (µM) varied greatly depending on the metabolite measured. For example, the amount of urea in the urine is log orders greater in magnitude compared to lactate. To appreciate the impact of a lower concentration metabolite in the model, equal to the change in an abundant metabolite, the methods must detect the relative change in concentration. Thus, metabolite concentrations were mean-centered followed by unit variance scaling (or z-scoring). Thereafter, partial least squares discriminant analysis (PLS-DA) was performed (SIMCA-P 11). PLS-DA determines the relationship between the response vector Y (i.e. subject group) and the matrix X (concentration of each metabolite) by simultaneous projections of both Y and X spaces to a plane. Then seven fold internal crossvalidation was performed by dividing the data into seven parts (by default) and each1/7^{th} in turn was removed. A final model was chosen based on the 6/7^{th} metabolite data left in the new model. This process identifies the metabolites whose concentrations differed significantly between groups of animals or patients, seen as a coefficient of variation plot (Figure 1B). Initially, a model with all variables was created. The greater the consistent difference in metabolite concentration between groups, the more important the metabolite became in creating the model, seen as a Variables of Importance plot (VOI, Figure 1C). Nonparametric testing was also performed on the metabolite values to cross check the metabolites of importance generated by SIMCA (Mann Whitney test, Statview 4.5). The final list of metabolites in the Results section was chosen based on the VOI and the confirmation of statistical significance by nonparametric analysis. This procedure resulted in the most accurate model, both with respect to correlation coefficients (R2) and prediction properties (Q2).

### Separation of Animal Groups Based on the XY-trace of the NMR Spectral Data:

1D ¹H-NMR spectra for each animal's sample (32k data points) was exported using the Varian macro xy-trace (ASCII format) for multivariate statistical analysis. The program converts the visual display of the 1-D spectrum line to a series of values in terms of position along the X and Y axis. For example, for each position on the X axis, there is a value of Y representing the peaks and troughs seen on the NMR tracing. The value for Y at a given point X are created by the different types and concentration of metabolites in the urine. Thus, the xy-trace data also characterize and quantify the metabolites but only terms of their position on the xy-data; the metabolites do not have to be identified^{27, 28}. Six pairs of classifiers were created: control vs. challenged, sensitized vs. challenged, control vs. sensitized, control vs. challengeddex, challenged vs. challengeddex, and sensitized vs. challengeddex (see Table 4). To separate each pair of classifiers, values of Y for each 0.04 section of width on the x axis were calculated and compared using a genetic-algorithm-based feature selection approach²⁹. The feature selection was wrapper-based: i.e. a classifier, in this case linear discriminant analysis (LDA) with leave-one-out (LOO) internal cross validation was used to identify optimal features. The unique combination of regions within the spectra, which could separate the pairs of classifiers, became the feature selection. Having obtained the optimal feature set for each pair of classifiers, external cross validation (EXCV) was performed to estimate a more realistic error rate. Each dataset was split randomly into a training set and a monitoring test set (50:50). The splitting was stratified (i.e. the relative proportions of the samples in the two classes were retained in the splits). Ten splits were done for each dataset and the averages and standard deviations were calculated. Based on experience with small sample sizes, this approach was used to give a more realistic result than using the entire dataset^{27, 28}.

This method does not directly identify metabolites. To identify the metabolites, one would have to look at the regions of interest suggested as relevant by the analysis above and then characterize plausible metabolites that could create the xy-values. Despite this shortcoming, the xy-trace method has advantages. It adds to the known metabolite data, as it incorporates all values measured by NMR, even as yet uncharacterized metabolites. Thus, if efforts are made to identify metabolites directly, this may identify metabolites not previously considered that could have robust effects in differentiating NMR spectra. Further, a computer could use these values of Y at position X with greater ease. There is no need for an operator to measure values as in the known metabolite method as the metabolites do not need to be identified. For example, if there are 5 regions on the x axis that are relevant for differentiating between asthma and non asthma, a computer based program could quickly focus on the values of Y at these regions and based on the model, suggest the chance that the NMR data reflects disease or not. The xy-trace data, then has a great advantage for its speed and operator independence.

### Results

***The animal model of asthma exacerbation:*** Although the response to histamine in sensitized GP was similar to control animals, the challenged group demonstrated increased reactivity with significantly higher bronchoconstriction responses compared with control (P<0.0001). Challengeddex showed diminished response compared to challenged alone (p<0.05). While there was no significant difference between total cell counts of the control, sensitized, or controldex groups, total cells in the lung lavage of challenged were elevated (p <0.0001 each), shown in Figure 2B. Total and differential leukocyte counts were performed on the lung lavage of control (white bars), sensitized (hatched bars), challenged (black bar), challengeddex (grey bars), and controldex animals (cross bars), (n=9 each, B). The increase consisted largely of lymphocytes, eosinophils and neutrophils. Macrophage values were similar among all groups except the controldex group, which was higher compared with control (p=0.03). Total cells in challengeddex remained higher similar to challenge, except for lymphocytes that were significantly lower (p<0.05). Sensitized animals showed higher eosinophil count than controls (P<0.0001), shown in Figure 2C. Eosinophils were counted in the cartilaginous airways of control (white bars), sensitized (hatched bars), challenged (black bar), and challengeddex (grey bars), (n=12 each, C). Challenge of sensitized animals further increased the eosinophil count compared with the control (P<0.0006) and sensitized animals (P<0.0001). Eosinophils in the challengeddex group remained similar to challenged.

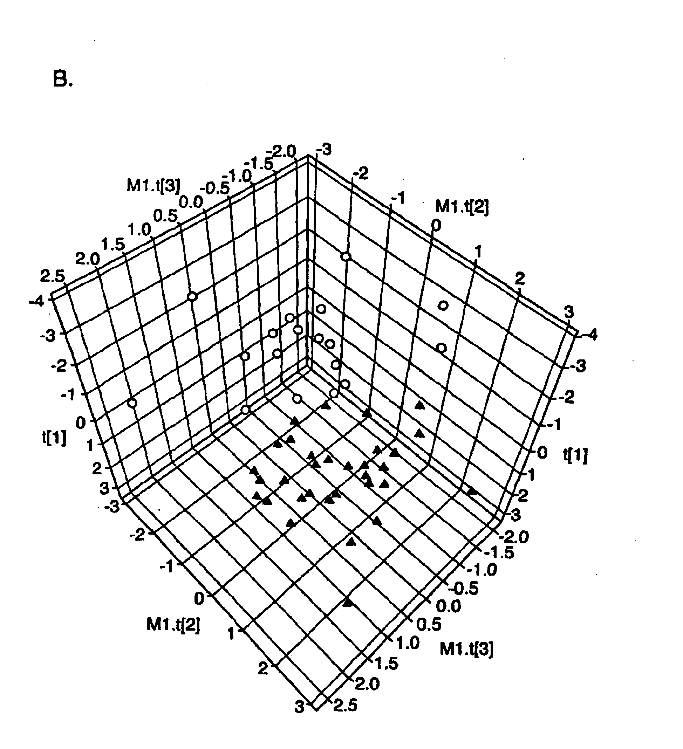

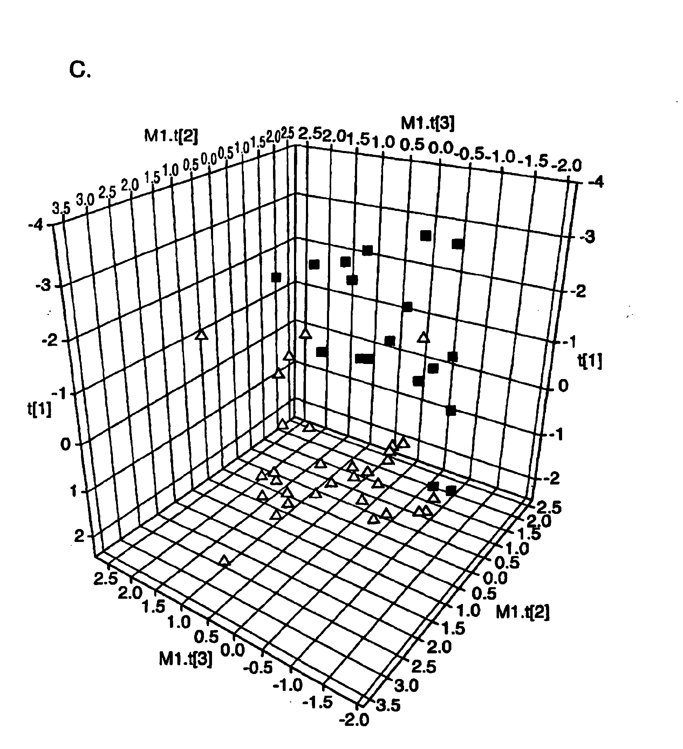

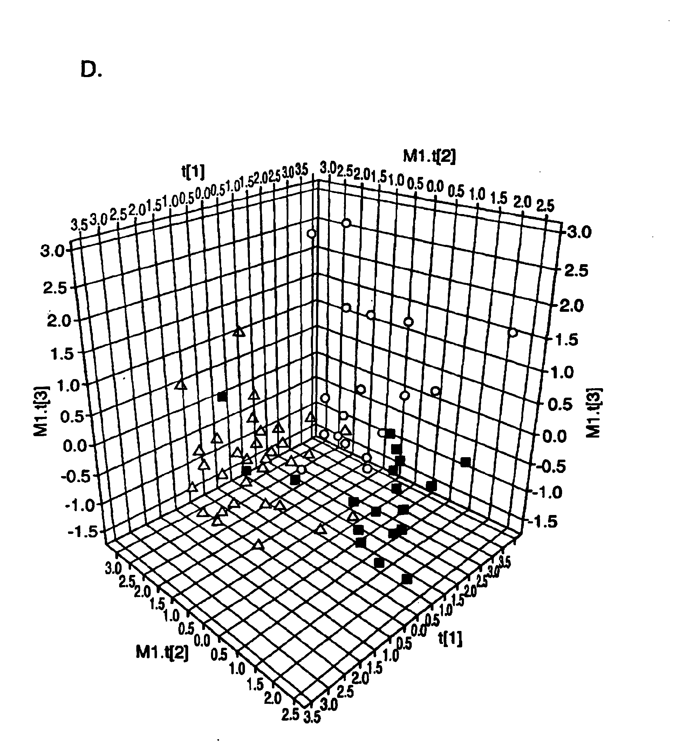

***Analysis of animal model using known metabolite values:*** Targeted NMR metabolite profiling can differentiate groups of animals: Using a library of known metabolite standards (Chenomx), the concentrations of 50 metabolites were measured in the urine of all animals, shown in Figure 1A, and each group was compared using PLS-DA. Not all metabolites were required to separate the groups and in some cases adding metabolites made the model accuracy worse. The final list of metabolites (and their concentrations) used in each separation model were based on the PLS-DA VOI ranking and nonparametric analysis (Table 1). The differences in concentration of metabolites between groups are shown as the Coefficient of Variation plots Figures 3A-C. Separation of each pair of animal groups and the 3-way comparison of groups is illustrated in Figure 4 (A: control (circles) vs. sensitized (squares), R²= 0.53 Q²=0.29); B: control vs. challenged (triangles), R²= 0.74 Q²=0.59; C: sensitized vs. challenged, R²=0.63 Q²=0.50; and D: 3-way analysis of animal groups (R²=0.54 Q²=0.25). As expected, dexamethasone induced an altered urine metabolite profile, as determined by PLS-DA analysis, shown in Figure 5A and B, challenged vs. challengeddex: R²=0.76 Q²=0.44; control vs. controldex: R²=0.83 Q²=0.69 (data not shown). Despite the small sample size, an accuracy measurement was determined using PLS-DA generated models. Fifteen urine samples from animals not part of the original PLS-DA modeling were run blindly (sensitized, n=4, challenged, n=7, challengeddex n=4). The models created by PLS-DA correctly categorized challenged animals 19 of 21 tests (accuracy of 90%); sensitized 5 of 8 tests (accuracy of 62%) and challenegeddex correctly 3 of 4 (75% accuracy).

***Analysis of Children with asthma using known metabolite values:*** Pediatric patients attending an outpatient assessment clinic (chronic stable asthma patients) were recruited for urine sampling. In addition, children in the emergency department (exacerbated asthma patients) with an acute exacerbation of asthma provided a urine sample for NMR spectroscopy. These were compared to age and sex match control without any chronic or acute illness (Table 2). Using known metabolites, PLS-DA analysis of the urine revealed metabolite concentrations that were significantly different between groups. The graphical patient separation is shown in Figures 6-9. This separation is based on differences in concentration of metabolites between groups, shown as the Coefficient of Variation plots. The final list of metabolites used to separate each pairing or the 3-way comparison (as shown in Table 3) were based on the PLS-DA Variables of Importance (VOI) ranking within the model. Removing the least important metabolites, it was determined that the best model of separation of asthma outpatients (ie. chronic stable asthma patients) versus healthy control used the top 23 metabolites (Figure 6(c)). The model had a correlation coefficient of R2=0.72 and a predictive index of Q2=0.67. The best separation model (R2=0.78, Q2=0.72) of healthy controls vs. asthma patients in the emergency department (ie. those with exacerbated asthma) was determined using the top 20 metabolites in the VOI list (Figure 7(c)). Figures 8A and 8B illustrate data for asthma patients in the outpatient clinic (ie. chronic stable asthma patients) vs. asthma patients in the emergency department (exacerbated asthma patients) (R2=0.84, Q2=0.74), and the top 28 metabolites in that VOI list were used to provide the separation model. Finally, figure 9 comparing all three groups (R2=0.74, Q2=0.61), required the top 30 metabolites in its respective VOI list. These metabolites considered as most important for at least one of the separation models are listed with their concentrations in Table 3.

To determine the real world accuracy of this model and its applicability as a diagnostic tool for asthma in the outpatient clinic, the concentrations of metabolites from outpatient asthma children (n=33) not originally part of the modeling were entered into the model. These values were entered into the computer without a diagnosis, thus the computer was blinded for the PLS-DA derived model of outpatient asthma versus control. The model was able to diagnose the blinded asthma samples with 94% accuracy (31 correct of 33 samples). Blinded samples from healthy control children assessed by the model were correctly classified in 19/20 samples. Thus, the model had a 5% (1/20) rate of misclassification or false positives for healthy control children.

For the separation of asthma outpatient versus those with asthma in the ED, it is important to note that while many of these metabolites were also part of the outpatient asthma versus control model, new metabolites were needed for the separation of asthma in the ED. To determine the real world accuracy of this model and its applicability as a diagnostic tool for impending asthma exacerbation in the outpatient clinic, the concentrations of metabolites from outpatient asthma children (n=33) not originally part of the modeling were entered into the model. These values were entered into the computer without a diagnosis, thus the computer was blinded for the PLS-DA derived model of outpatient asthma versus control. The model was able to diagnose the blinded asthma samples with 91% accuracy (30 correct of 33 samples). Insufficient asthma exacerbation children subjects were available to perform blinded analysis as yet.

As might be expected, especially given the small population size, the 3-way model was unable to give the same degree of accuracy compared to the 2-way models. The blinded outpatient asthma samples were only diagnosed 22 correctly of 33 samples (66% accuracy) and healthy controls correctly 13 of the 20 (a 35% false positive rate).

***Analysis of animal model using xy-trace data:*** The xy-trace of NMR spectra can also differentiate guinea pig models: NMR spectra exported as xy-trace were analyzed using the feature selection component (with LDA/LOO internal cross validation) of the Statistical Classification Strategy (54). From these, spectral regions features were determined that could separate the different animal populations, shown in Figure 10A. To determine an estimate of separation accuracy using these regions, external cross-validation (EXCV), using ten 50:50 random splits, was performed. EXCV confirmed the ability of xy-trace data analysis to separate, in a pair-wise fashion, the different groups of animals with the average accuracies and standard deviations (SD) presented in Table 4. For example, the control vs. challenged groups could be discriminated with a minimal accuracy of 80.4 ± 5.9%, in the training set (TR) and 82.6 ± 6.9% in the monitoring set (MO). The ability to separate groups is illustrated using Relative Distance Plan mapping, shown in Figure 10B-D. Overall, based on xy-trace data, NMR spectral regions could discriminate between the populations with a minimal accuracy of 80%, with some discrimination occurring with greater than 90% accuracy.

**Table 1: Concentrations of metabolites (mmol/mmol creatinine), which were used to discriminate the different guinea pig groups; shown as mean (STD)**

| **Metabolites** | **Control (n=18)** | **Sensitized (n=20)** | **Challenged (n=32)** | **Control/Dex (n=4)** | **Challenged/Dex (n=12)** |
|---|---|---|---|---|---|
| 1-Methyl-Histamine | 0.050(0-0.097) | 0.052(0.012-0.076) | 0.093(0.046-0.113) | 0.015(0.008-0.023) | 0.044(0.002-0.055) |
| 2-OH-Isobutyrate | 0.051(0.031-0.065) | 0.050(0.040-0.064) | 0.028(0.020-0.044) | 0.043(0.038-0.045) | 0.043(0.031-0.061) |
| 2-Oxoglutarate | 0.011(0.007-0.017) | 0.012(0.008-0.016) | 0.010(0.007-0.014) | 0.004(0.003-0.005) | 0.006(0.005-0.012) |
| 3-OH-3-Methyl-Glutarate | 0.014(0.008-0.025) | 0.012(0.007-0.018) | 0.010(0.007-0.015) | 0.004(0.003-0.005) | 0.010(0.006-0.014) |
| 3-OH-Butyrate | 0.037(0.028-0.044) | 0.041(0.021-0.051) | 0.021(0.010-0.034) | 0.041(0.029-0.048) | 0.018(0.013-0.029) |
| 3-Methyl-Adipate | 0.025(0.021-0.032) | 0.025(0.017-0.029) | 0.014(0.009-0.022) | 0.015(0.008-0.018) | 0.011(0.009-0.017) |
| 4-Aminohippurate | 0.010(0.010-0.020) | 0.005(0-0.010) | 0.010(0.010-0.010) | 0.090(0.040-0.115) | 0.010(0.005-0.010) |
| 4-OH-Phenylacetate | 0.144(0.114-0.191) | 0.121(0.094-0.167) | 0.126(0.081-0.157) | 0.101(0.095-0.130) | 0.122(0.068-0.196) |
| 4-Pyridoxate | 0.010(0.006-0.014) | 0.004(0.002-0.009) | 0.009(0.007-0.010) | 0.004(0.003-0.005) | 0.009(0.005-0.010) |
| Acetate | 0.266(0.198-0.535) | 0.212(0.136-0.450) | 0.170(0.108-0.210) | 0.137(0.098-0.1880) | 0.118(0.081-0.203) |
| Acetoacetate | 0.010(0.009-0.013) | 0.013(0.007-0.018) | 0.014(0.010-0.020) | 0.011(0.009-0.012) | 0.007(0.005-0.010) |
| Acetone | 0.003(0.002-0.007) | 0.004(0.003-0.007) | 0.007(0.005-0.010) | 0.004(0.003-0.005) | 0.005(0.003-0.009) |
| Adenosine | 0(0-0.004) | 0(0-0.002) | 0(0-0.005) | 0.002(0-0.003) | 0(0-0.001) |
| Carnitine | 0.020(0.020-0.030) | 0.015(0.010-0.020) | 0.010(0.010-0.020) | 0.140(0.120-0.175) | 0.010(0.010-0.015) |
| Creatine | 0.036(0.026-0.049) | 0.034(0.026-0.050) | 0.022(0.014-0.041) | 0.039(0.035-0.045) | 0.026(0.016-0.033) |
| Dimethylamine | 0.101(0.082-0.119) | 0.100(0.083-0.118) | 0.115(0.102-0.143) | 0.096(0.087-0.110) | 0.122(0.103-0.146) |
| Fumarate | 0(0-0.010) | 0(0-0) | 0(0-0.005) | 0(0-0) | 0(0-0) |
| Glucose | 0.148(0.086-0.238) | 0.155(0.121-0.264) | 0.106(0.071-0.143) | 0.121(0.117-0.137) | 0.155(0.100-0.334) |
| Glycolate | 0.010(0-0.016) | 0(0-0) | 0(0-0.015) | 0(0-0) | 0(0-0) |
| Isobutyrate | 0(0-0) | 0(0-0) | 0(0-0) | 0(0-0.50) | 0(0-0) |
| Kynurenate | 0.010(0.006-0.015) | 0.007(0.004-0.014) | 0.009(0.005-0.013) | 0.005(0.004-0.010) | 0.006(0.004-0.008) |
| Malonate | 0.014(0.011-0.020) | 0.014(0.013-0.021) | 0019(0.014-0.021) | 0.016(0.011-0.020) | 0.014(0.010-0.018) |
| Methylamine | 0.018(0.013-0.040) | 0.043(0.010-0.049) | 0.028(0.014-0.042) | 0.009(0.009-0.014) | 0.033(0.026-0.069) |
| Myolnositol | 0.040(0.030-0.060) | 0.040(0.030-0.060) | 0.040(0.030-0.055) | 0.410(0.380-0.570) | 0.035(0.020-0.080) |
| Oxalacetate | 0.250(0.185-0.287) | 0.197(0.157-0.264) | 0.154(0.117-0.220) | 0.237(0.228-0.247) | 0.249(0.197-0.323) |
| Phenylacetylglycine | 0.156(0.113-0.196) | 0.115(0.094-0.152) | 0.121(0.083-0.153) | 0.024(0.022-0.026) | 0.140(0.095-0.213) |
| Phenylalanine | 0.043(0.035-0.079) | 0.046(0.029-0.058) | 0.041(0.036-0.059) | 0.011(0.009-0.013) | 0.022(0.016-0.042) |
| Sarcosine | 0.013(0.009-0.018) | 0.013(0.005-0.017) | 0.009(0.004-0.031) | 0.004(0.003-0.005) | 0.010(0.007-0.016) |
| Succinate | 0.024(0.018-0.073) | 0.036(0.022-0.058) | 0020(0.009-0.046) | 0.013(0.011-0.014) | 0.006(0.005-0.008) |
| Succinylacetone | 0.014(0.009-0.021) | 0.015(0.012-0.021) | 0.013(0.009-0.018) | 0.004(0.003-0.005) | 0.011(0.009-0.020) |
| Trigonelline | 0.073(0.050-0.086) | 0.083(0.064-0.104) | 0.061(0.039-0.080) | 0.053(0.044-0.059) | 0.069(0.063-0.091) |
| Trimethylamine | 0(0-0) | 0(0-0) | 0(0-0) | 0(0-0) | 0(0-0) |
| Trimethyl-N-Oxide | 0.037(0.021-0.057) | 0.056(0.043-0.079) | 0.045(0.018-0.077) | 0.089(0.076-0.107) | 0.045(0.027-0.062) |
| Tyrosine | 0.043(0.062-0.033) | 0.035(0.027-0.049) | 0.035(0.023-0.050) | 0.027(0.016-0.042) | 0.024(0.016-0.032) |

**TABLE 2 Characteristics of children enrolled in the study**

| | Asthma Outpatients | Asthma Emergency Department | Healthy Controls |
|---|---|---|---|
| | (n=73) | (n=20) | ( n=42) |
| Median Age Range (years) | 8.08 (3-13) | 5.05 (2-14) | 8.38 (4-13) |
| Sex (Male/Female) | 21/11 | 10/10 | 18/14 |
| Atopy Status (Yes/No) | 25/7 | 13/7 | N/A |
| % predicted FEV₁ | | | |
| Mean, SD | 85.08, 16.88 | N/A | N/A |
| ICS Use (Yes/No) | 23/9 | 9/11 | N/A |

| | | | |
|---|---|---|---|
| Note: FEV₁: Forced expiratory volume at 1 second; ICS: Inhaled corticosteroids; N/A = not applicable/available. **ICS: Inha** | | | |

**TABLE 3. The concentration of each metabolite used to discriminate the different groups of children. (α) - required for separation of Outpatient Asthma vs. Control; (β) - required for separation of Outpatient Asthma vs. ED Asthma; (γ) - required for separation of Outpatient Asthma vs. ED Asthma vs. Control. Shown are median and interquartile ranges (IQR) in mmol of metabolite/mmol creatinine.**

| Metabolites | Healthy Controls | Outpatient Asthma | Asthma in ED | Outpatient Asthma Blinded |
|---|---|---|---|---|
| 1-MethylHistamine (α,β,γ) | 0.003 (0.000, 0.006) | 0.017 (0.008, 0.049) | 0.007 (0.002, 0.010) | 0.018 (0.009, 0.038) |
| 1-Methylnicotinamide (α,β,γ) | 0.005 (0.003, 0.007) | 0.012 (0.008, 0.017) | 0.005 (0.004, 0.008) | 0.011 (0.005, 0.016) |
| 2-Hydroxybutyrate | 0.001 (0.001, 0.002) | 0.002 (0.001, 0.002) | 0.003 (0.002, 0.004) | 0.003 (0.001, 0.004) |
| 2-Hydroxyisobutyrate (α) | 0.006 (0.005, 0.007) | 0.007 (0.006, 0.008) | 0.010 (0.010, 0.010) | 0.007 (0.007, 0.010) |
| 2-Methylglutarate (β,γ) | 0.010 (0.009, 0.012) | 0.011 (0.009, 0.015) | 0.015 (0.010, 0.040) | 0.012 (0.011, 0.014) |
| 2-Oxoglutarate (α,β,γ) | 0.015 (0.011, 0.024) | 0.005 (0.004, 0.009) | 0.018 (0.012, 0.029) | 0.012 (0.005, 0.021) |
| 3-OH-3-Methylglutarate (β,γ) | 0.004 (0.003, 0.005) | 0.003 (0.003, 0.005) | 0.005 (0.004, 0.007) | 0.004 (0.004, 0.006) |
| 3-Methyladipate (α, β,γ) | 0.002 (0.001, 0.003) | 0.002 (0.002, 0.003) | 0.005 (0.003, 0.008) | 0.003 (0.002, 0.005) |
| 3-Hydroxybutyrate | 0.010 (0.006, 0.015) | 0.011 (0.008, 0.019) | 0.023 (0.009, 0.055) | 0.014 (0.010, 0.019) |
| 4-Aminohippurate (α,β,γ) | 0.001 (0.000, 0.002) | 0.001 (0.001, 0.002) | 0.000 (0.000, 0.002) | 0.001 (0.001, 0.003) |
| 4-Pyridoxate (α) | 0.001 (0.001, 0.002) | 0.001 (0.000, 0.002) | 0.003 (0.002, 0.004) | 0.002 (0.001, 0.003) |
| Acetone (β,γ) | 0.003 (0.002, 0.003) | 0.019 (0.015, 0.023) | 0.007 (0.003, 0.043) | 0.003 (0.002, 0.004) |
| Adenine (α,β) | 0.000 (0.000, 0.000) | 0.000 (0.000, 0.000) | 0.000 (0.000, 0.000) | 0.000 (0.000, 0.002) |
| Adenosine (α,γ) | 0.000 (0.000, 0.000) | 0.000 (0.000, 0.001) | 0.001 (0.000, 0.001) | 0.000 (0.000, 0.001) |
| Alanine (β,γ) | 0.030 (0.024, 0.043) | 0.035 (0.028, 0.044) | 0.059 (0.041, 0.124) | 0.043 (0.032, 0.058) |
| Carnitine (α,γ) | 0.011 (0.006, 0.017) | 0.007 (0.002, 0.016) | 0.015 (0.008, 0.059) | 0.008 (0.004, 0.014) |
| Creatine (γ) | 0.104 (0.074, 0.141) | 0.203 (0.050, 0.417) | 0.219 (0.132, 0.460) | 0.183 (0.075, 0.323) |
| Dimethylamine (β,γ) | 0.034 (0.032, 0.038) | 0.039 (0.032, 0.043) | 0.054 (0.045, 0.066) | 0.040 (0.034, 0.048) |
| Formate (β,γ) | 0.029 (0.021, 0.049) | 0.043 (0.027, 0.057) | 0.045 (0.029, 0.055 | 0.041 (0.026, 0.052) |
| Fumarate (β,γ) | 0.000 (0.000, 0.001) | 0.000 (0.000, 0.001) | 0.002 (0.001, 0.007) | 0.001 (0.000, 0.002) |
| Glucose (β,γ) | 0.039 (0.036, 0.047 | 0.041 (0.032, 0.053) | 0.092 (0.035, 0.590) | 0.048 (0.031, 0.073) |
| Glycine | 0.118 (0.082, 0.152) | 0.123 (0.100, 0.169) | 0.160 (0.113, 0.224) | 0.145 (0.110, 0.207) |
| Glycolate (β,γ) | 0.053 (0.050, 0.064) | 0.024 (0.016, 0.042) | 0.077, (0.064, 0.106) | 0.057 (0.033, 0.083) |
| Hippurate (α) | 0.158 (0.065, 0.241) | 0.126 (0.083, 0.239) | 0.200 (0.091, 0.348) | 0.227 (0.087, 0.365) |
| Histidine (γ) | 0.048 (0.036, 0.078) | 0.028 (0.000, 0.062) | 0.000 (0.000, 0.010) | 0.009 (0.000, 0.043) |
| Homovanillate (α,γ) | 0.002 (0.002, 0.003) | 0.003 (0.002, 0.005) | 0.017 (0.000, 0.085) | 0.004 (0.002, 0.007) |
| Imidazole (β) | 0.000 (0.000, 0.000) | 0.019 (0.000, 0.253) | 0.000 (0.000, 0.000) | 0.000 (0.000, 0.000) |
| Kynurenine (α,γ) | 0.001 (0.000, 0.002) | 0.002 (0.001, 0.003) | 0.002, (0.000, 0.003) | 0.002 (0.000, 0.003) |
| Lactate (β) | 0.024 (0.021, 0.034) | 0.026 (0.021, 0.037) | 0.034 (0.027, 0.047) | 0.028 (0.020, 0.044) |
| Methylamine (α,β) | 0.003 (0.001, 0.003) | 0.002 (0.001, 0.002) | 0.002 (0.001, 0.003) | 0.002 (0.001, 0.003) |
| Myo-Inositol (β,γ) | 0.009 90.005, 0.009) | 0.010 (0.009, 0.015) | 0.032 (0.013, 0.049) | 0.013 (0.009, 0.022) |
| O-Acetylcarnitine (α,β,γ) | 0.006 (0.004, 0.008) | 0.004 (0.002, 0.005) | 0.019 (0.009, 0.046) | 0.005 (0.003, 0.007) |
| Oxaloacetate (β) | 0.068 (0.057, 0.098) | 0.069 (0.042, 0.084) | 0.078 (0.069, 0.144) | 0.071 (0.059, 0.095) |
| Phenylacetylglycine (β,γ) | 0.027 (0.022, 0.042) | 0.073 (0.042, 0.104) | 0.008 (0.005, 0.010) | 0.031 (0.020, 0.080) |
| Phenylalanine (α,β,γ) | 0.004 (0.003, 0.005) | 0.010 (0.007, 0.013) | 0.005 (0.004, 0.007) | 0.010 (0.008, 0.016) |
| Succinate (α,γ) | 0.015 (0.007, 0.018) | 0.026 (0.016, 0.041) | 0.039 (0.020, 0.065) | 0.025 (0.019, 0.040) |
| Taurine (γ) | 0.059 (0.032, 0.109) | 0.076 (0.029, 0.113) | 0.072 (0.036, 0.123) | 0.056 (0.026, 0.108) |
| Threonine (α,γ) | 0.012 (0.008, 0.015) | 0.006 (0.005, 0.007) | 0.006 (0.005, 0.011) | 0.008 (0.005, 0.011) |
| Trigonelline (α) | 0.004 (0.003, 0.007) | 0.005 (0.002, 0.009) | 0.005 (0.002, 0.012) | 0.005 (0.002, 0.008) |
| Trimethylamine N-oxide (α,γ) | 0.050 (0.037, 0.075) | 0.032 (0.020, 0.045) | 0.029 (0.019, 0.051) | 0.040 (0.020, 0.065) |
| Tryptophan (α,β,γ) | 0.004 (0.003, 0.006) | 0.005 (0.003, 0.009) | 0.004 (0.003, 0.007) | 0.007 (0.003, 0.010) |
| Tyrosine (α,β) | 0.010 (0.008, 0.015) | 0.010 (0.008, 0.014) | 0.015 (0.010, 0.019) | 0.012 (0.009, 0.017) |
| Yellow-7.1 (α) | 0.000 (0.000, 0.005) | 0.004 (0.002, 0.016) | 0.000 (0.000, 0.019) | 0.000 (0.000, 0.006) |
| cis-Aconitate (β,γ) | 0.000 (0.000, 0.000) | 0.000 (0.000, 0.000) | 0.006 (0.003, 0.009) | 0.000 (0.000, 0.007) |
| trans-Aconitate (β) | 0.004 (0.003, 0.005) | 0.005 (0.003, 0.006) | 0.004 (0.003, 0.007) | 0.004 (0.003, 0.006) |

**TABLE 4: Group separation accuracy by optimal NMR spectral region analysis**

| Pair Classifiers | Optimal regions (PPM) | Training sets | | | Tests sets | | |
|---|---|---|---|---|---|---|---|
| | | Acc(1) | Acc(2) | Acc(O) | Acc(1) | Acc(2) | Acc(O) |
| Control vs. Sensitized | 3.93-3.92 | 82.5± 7.3 | 88.2± 8.6 | 85.2± 6.6 | 85.8± 9.7 | 88.4± 11.0 | 87.1± 6.0 |
| | 3.71-3.72 | | | | | | |
| | 1.10-1.12 | | | | | | |
| Control vs. Challenged | 3.56-3.58 | 76.7± 12.0 | 83.6± 9.6 | 80.4± 5.9 | 81.7± 14.0 | 83.3± 14.0 | 82.6± 6.8 |
| | 3.33-3.34 | | | | | | |
| | 2.91-2.94 | | | | | | |
| Sensitized vs. Challenged | 3.92-3.94 | 83.6± 15.0 | 87.2± 9.4 | 85.6± 6.3 | 81.2± 21.0 | 88.7± 12.0 | 85.6± 7.5 |
| | 3.20-3.22 | | | | | | |
| | 2.25-2.27 | | | | | | |
| | 2.11-2.14 | | | | | | |
| Challenged vs. Challengeddex | 3.88-3.92 | 87.2± 8.8 | 85.6± 5.4 | 86.6± 4.8 | 87.9± 12.0 | 80.0± 19.0 | 84.6± 6.2 |
| | 3.44-3.48 | | | | | | |
| | 3.16-3.20 | | | | | | |
| Control vs. Challengeddex | 4.18-4.21 | 91.7± 7.9 | 87.8± 8.2 | 90.0± 2.7 | 96.7± 5.8 | 91.1± 13.0 | 94.3± 4.9 |
| | 2.69-2.72 | | | | | | |
| | 2.39-2.41 | | | | | | |
| Sensitized vs. Challengeddex | 2.91-2.93 | 88.2± 6.1 | 94.5± 7.9 | 91.0± 5.2 | 93.3± 9.5 | 90.0± 11.0 | 91.9± 6.7 |
| | 1.80-1.80 | | | | | | |
| | 1.65-1.65 | | | | | | |

Optimal NMR spectral regions for separation of the guinea pig populations was determined by linear discriminant analysis and leave-one-out internal cross validation. Training sets show the average and standard deviations of the classification result after external cross validation, using 10 random splits of the data. Test sets shows the predictive discriminative accuracy for these regions determined after NMR data from animals not part of the original training set was blindly tested using the model. Ac(1), Ac(2) and Ac(O) refer to class 1, class 2 and overall percent classification accuracies.

### Analysis and Discussion of Results

It has become evident that the current methods used to follow patients in most asthma clinics (i.e. using history, physical, and spirometry) are somewhat insensitive in their ability to reliably identify changes in airway pathophysiology necessitating therapeutic intervention^{6,30}. Furthermore, spirometry, the most common objective measurement available to a majority of physicians, is not feasible for most young children less than 6 years of age. A Cochrane analysis suggested that measurement of inflammatory changes improves clinical outcomes³¹. While noninvasive tests, like sputum analysis or exhaled NO, have shown clinical utility, each has some limitations³². Sputum is not practical for all patients, especially the very young. Exhaled NO is easier to perform, but its diagnostic utility appears less than sputum analysis. In addition, both tests rely on essentially one biomarker (i.e. eosinophilia or NO, respectively) to describe the heterogeneous phenotype of asthma. Experience with NMR analysis of sputum in patients with cystic fibrosis led us to consider urine as another biofluid for NMR analysis³³.

The animal model described herein is an established animal model of allergic airway dysfunction, which in part reflects what occurs during a human asthma exacerbation. Airway reactivity is commonly measured as the degree of bronchoconstriction in response to a variety of agents, including histamine¹⁸. As expected, antigen challenged animals developed AHR, which correlated with increased airway inflammation measured in lung fluid and histology. Airway inflammation is a complex physiological state involving the metabolism of not only the inflammatory cells, but the cells affected by inflammation, including epithelium, smooth muscle, nerves, and connective tissue. In addition it is important to consider that asthma is a systemic illness with cell recruitment from bone marrow, through blood to lung tissue³⁴. Further, the stress of airway dysfunction could affect organs outside the lung (i.e. hypoxemic brain injury). Therefore the methodology described herein is designed to consider a wider range of biomarkers than just those associated with classic airway inflammation. The evidence that it works in human subjects is also compelling.

Multivariate statistical analysis of the NMR spectra, using either the metabolite concentrations or spectral data exported as a xy-trace could discriminate between the various groups in the models representing the spectrum of an asthma exacerbation. It is important to note that while most of the regions determined from xy-trace data represent largely as yet unknown metabolites, there was crossover between the methodologies. For example, of the regions shown in Figure 10A, metabolites such as oxalacetate, glucose, and tyrosine have resonant peaks in these areas and could be one of the metabolites being detected by the xy trace/statistical method. Thus, the two methodologies for spectral and metabolomic analysis become complementary by identifying expected and unexpected metabolites in NMR spectra. On-going research is characterizing the relevant new metabolites identified by xy trace data and multivariate analysis.

The study identified groups of metabolites which are useful as marker metabolites in discriminating between chronic stable asthma and a non-disease state (without asthma) in humans, between chronic stable asthma and exacerbated asthma in humans, and between chronic stable asthma, exacerbated asthma, and a non-disease state in humans.

In this respect, a method of diagnosing an asthma disease state in a subject is provided. The method is defined in claim 1 and allows to differentiate between chronic stable asthma and exacerbated asthma.

This study was not designed to confirm the importance of specific metabolic pathways resulting from airway dysfunction. However, based on the data, some speculations of possible relevant pathways have been developed, which could impact future study design. For example, 2-hydroxyisobutyrate, 3-hydroxybutyrate and 3-methyladipate were important. The later two metabolites are produced predominantly by the liver during β-oxidation³⁵. The reduction also correlated with a decrease in urine glucose. The difference observed in challenged guinea pigs may reflect a switch from fatty acid oxidation to glycolysis. These metabolites were also relevant in children with asthma.

Through a brief review of the guinea pig and the human studies it is evident a particular strength of the methods disclosed herein is the ability to identify metabolites that may be generally classified as markers of hypoxia. In the humans, an asthma exacerbation causes severe breathing difficulties. Such difficulty causes hypoxic stress on the person's body tissues. Even in outpatient settings, asthma patients have some airway obstruction, which theoretically could cause some degree of hypoxemic stress on a person's body tissues, albeit milder. The metabolites suggested from the challenged animals were also seen as relevant for the model of asthma exacerbation children in the ED compared to asthma patients in clinic or healthy control. Many of the metabolites identified above by the methods presented herein have been correlated in parallel investigations to be indicative of hypoxic insults. For example, metabolites related to the citric acid cycle appear to be effected. 2-oxaloglutarate was important in all models of separation and oxaloacetate appeared as a marker for children in the ED. Both have effects on hypoxic-inducible factors (HIF)³⁶, which constitute a large family of genes involved in energy metabolism, cell growth, angiogenesis, and erythropoiesis³⁷. 2-oxoglutarate was also required for the function of dioxygenases³⁸. With the present focus on respiratory diseases this correlation of multiple markers of hypoxemia is a unique characteristic of the disclosed methods.

The present study shows that succinate levels are higher in outpatient asthma compared to healthy controls, but this rises further in the ED asthma patients. Respiratory distress syndrome of infants was reported to affect urine levels of succinate^{39,40}. Fumarate is formed by the oxidation of succinate⁴¹ and was also elevated in the ED samples. Another metabolite elevated in the ED samples, 3-Hydroxy-3-Methylglutarate, is important in the production of succinate⁴¹ and contributes to oxidative damage⁴². Threonine, an essential amino acid involved in the citric acid cycle, was an important marker for outpatient asthma versus control. Finally, cis- and trans-aconitate were important markers for outpatient asthma compared to those patients enrolled in the ED. With the exception of cis and trans-aconitate, we previously documented similar citric acid cycle metabolites have previously been documented using an animal model of asthma⁴³.

Similar to the effect on the citric acid, many metabolites could be linked to alterations in glucose and lipid metabolism. 2-hydroxyisobutyrate, 3-hydroxybutyrate, 3-methyladipate, and glucose were all relevant to the separation models³⁵. O-acetylcarnitine and carnitine were relevant for all models of separation, and are known for their role in oxidative metabolism in mitochondrial⁴¹. Both are association with hypoxemic stress^{44, 45}. Adenine is required for the production of adenosine⁴¹. Adenosine is an endogenous purine nucleoside important in cellular energy metabolism. In response to cellular damage, levels of adenosine typically rise. In asthma, adenosine could be both proinflammatory for mast cell stimulation but also anti-inflammatory for other cell types⁴⁶.

Some metabolites seen only in the model differentiating stable versus unstable asthma in the ED could be related to prolonged exertion and stress on glucose production. For example, lactate is elevated during anaerobic exercise⁴¹. Acetone is formed after the production of ketone bodies, usually when stores of glucose are too low, and stores of oxaloacetate have been used up⁴¹. Further, when lactate is being produced, alanine levels are expected to rise. Alanine is part of gluconeogensesis, an attempt to create glucose when stores are low⁴¹. Another metabolite important for energy regulation at the cellular level is creatine. In the present study creatine was a marker of worsening asthma. Creatine is endogenously synthesized and is phosphorylated to phosphocreatine in muscle and a rise in concentration may indicate an physiological state that is energy depleted^{47, 48}. Finally, glycolate is also important in energy production of mitochondria through the formation of the intermediates⁴⁹.

Protein and amino acid metabolism can also change during disease. A derivative of the metabolite nicotinamide, 1-methylnicotinamide, was important. Reports suggest that nicotinamide is a protective factor against asthma exacerbation⁵⁰. Phenyalanine is an essential amino acid critical in the production of tyrosine and catecholamines like epinephrine⁴¹. Catacholamines are important for asthma patients considering their effects on airway constriction⁵¹, and homeostasis during physical and psychological stress^{52, 53}. Homovanillate levels in urine are used in the diagnosis of catecholamine secreting tumors⁵⁴. Another consideration may be the modification of free tyrosine residues by eosinophil activity in the tissues⁵⁵. Modified tyrosine residues, 3-chlorotyrosine, and 3-bromotyrosine, have been previously idenitified in sputum samples taken from cystic fibrosis (CF) and asthma patients respectively³³. Without wishing to be bound by theory, that urinary tyrosine could also relate to increased metabolic activity of eosinophils and/or neutrophils. Unfortunately, the levels of 3-chloro and 3-bromotyrosine are below the limit of NMR detection.

Many of the metabolites have known involvement in either eliciting an inflammatory response, or attempting to dampen on-going damage. 1-methylhistamine was important in all the models. It is a downstream metabolite from histamine and it is reported that the serum level of 1-methylhistamine is higher in asthma patients, rises acutely following an asthma attack, and is lowered by anti-allergy medications^{56, 57, 58}. The present study confirmed these studies using a less invasive urine test. Kynurenine is a product of tryptophan metabolism by the enzyme indoleamine 2,3, dioxygenase (IDO)⁵⁹. IDO activity is important for immune regulation in health and disease including allergy⁶⁰. 4-Pyridoxic acid, the catabolic product of vitamin B6 (pyridoxine)⁴¹, was decreased in the studied asthma patients compared to controls. Similar lower levels of the active form of vitamin B6 (pyridoxal phosphate) have been reported in adults with asthma^{61, 62}.

Methylamine and its derivative dimethylamine are simple organic compounds found in water treatment plants and are used in industry⁶³. Increased serum levels were also reported in a rat model of gastric damage from NSAID over use⁶⁴. Trimethylamine N-oxide is an oxidation product of trimethylamine. Both trimethylamine N-oxide and dimethylamine have been reported in elevated amounts in diabetics⁶⁵ and in an animal model of renal damage⁶⁶. Formate, also a organic compound, is involved in fermentation by bacteria⁶⁷. 4-aminohippurate and its derivative hippurate were elevated in asthma groups. Aminohippuric acid is often used as a sodium salt by the pharmaceutical industry. Overall, it is unclear why these metabolites were elevated in the models compared to healthy controls, but one additional possibility could be their relation to their use in pharmaceutical drug manufacturing⁶⁸. Potentially, this data could be reflecting the expected increased use of medications in these children as they try to prevent an ED visit. When trying to predict impending asthma attack such accurate data on medication use could be helpful as often patients do not correctly communicate the amount of rescue medications they are taking.

Myo-inositol is a secondary-messenger in cellular functions including inflammatory cells ^{69, 70}. Many inflammatory cascades make use of inositol as a secondary messenger and on-going research is trying to understand the interaction of various cytokines and the effects on intracellular metabolism. In premature infants with respiratory distress syndrome a decrease in myo-inositol was detrimental to lung maturation and the healing of lung disease. In the guinea pig model of asthma myo-inositol was lowest in the challenged and highest in the challengeddex animals. It appeared to be selectively elevated in the ED children. This may suggest improved fatty acid metabolism, altered inflammatory signaling, or a pulmonary healing process following steroid treatment.

Phenylacetylglycine decreased following sensitization, and declined further following challenge. The human equivalent of the rodent model's phenylacetylglycine is phenylacetylglutamine⁷¹. Phenylacetylglutamine levels increase in humans and rodents with syndromes of phospholipidosis⁷²⁻⁷⁴, impaired amino acid absorption, and states of increased gut absorption of phenylacetate⁷². In humans, gut permeability and microflora may influence the development of atopy and asthma⁷⁵. The decreased concentration of phenylacetylglycine in the urine of sensitized and challenged guinea pigs may signify the generalized systemic effects of the asthma exacerbation model on gastrointestinal function.

Sarcosine is an intermediate in the metabolism of choline to glycine and can be found in muscles and other tissues of the body. The link between sarcosine and choline (an important component of the neurotransmitter acetylcholine) has been studied for sometime, but more recently sarcosine has been investigated for its role in modulating neurotransmission, particularly in schizophrenic patients. As a type-1 glycine transporter inhibitor, which indirectly activates the NMDA receptor, sarcosine may play a role in synaptic plasticity. The increase in sarcosine concentration found in the challenged guinea pigs may correlate to increased neurotransmission, muscle activity, or altered neural function. The level of sarcosine in the challenged guinea pig decreased following treatment with steroid, again suggesting a possible link of AHR to altered neural and muscular function.

As expected, dexamethasone administration altered urinary measurements of metabolism in both control and challenged guinea pigs. Dexamethasone-treated controls showed significantly lower levels of phenylacetylglycine than control. However, challenged animals appear to have even lower phenylacetylglycine levels, which returned to control levels after dexamethasone treatment. This suggests that while dexamethasone may disrupt normal amino acid and phospholipid homeostasis in control animals, it may be counteracting a pathological pathway in the challenged animals^{72, 74}. Dexamethasone appeared to reverse the effects of challenge on urinary excretion of 2-hydroxyisobutyrate and glucose as levels rose in challengeddex animals.

However, because the present methods are able to classify different respiratory diseases, they can clearly identify not just key metabolites indicative of unique pathophysiological processes, but also captures an overall pathologic profile that allows for the diagnosis of each particular pulmonary disease. The methods return a metabolite and biomarker profile that correlates to the basic physiological response of the animal and human subject, to the unique profile and metabolites produced by the pathophysiological pathways underlying the diagnosis and prognostic analysis.

A tremendous strength of the methods disclosed herein is the ability to detect, quantify, and follow these metabolites during unique physiological transitions that indicate states of disease and repair. The present studies demonstrate that airway dysfunction in an animal model of asthma correlates with an altered urinary NMR metabolite profile. The present studies show that ¹H- NMR spectroscopic analysis of urine in an animal model of asthma can differentiate animals with or without airway inflammation and airway hyperreactivity (AHR). There is provided a method for differentiating animals based on a combination of more than one metabolite, for example a "panel" or profile of biomarkers. These data have been extended successfully to humans with asthma.

There is increasing interest in the use of metabolomic based techniques in human disease⁷⁶. In the field of asthma, groups have studied single metabolites in a variety of biofluids including urine with variable success in clinical practice⁷⁷⁻⁸⁰. There is provided herein a method that uses multivariate statistical analysis on a combination of factors in urine that is novel in the field of asthma. There is provided herein a diagnostic method and tool, using urine NMR analysis, for monitoring humans with asthma, especially in children for whom most objective measures of airway function are often difficult.

While this invention has been described with reference to illustrative embodiments and examples, the description is not intended to be construed in a limiting sense. Thus, various modification of the illustrative embodiments, as well as other embodiments of the invention, will be apparent to persons skilled in the art upon reference to this description. It is therefore contemplated that the appended claims will cover any such modifications or embodiments.

### REFERENCES

1. Nadel JA, J.F.Murray, J.A.Nadel. Textbook of Respiratory Medicine: W.B. Saunders; 2000.
2. Kim CK, Chung CY, Choi SJ, Kim DK, Park Y, Koh YY. Bronchoalveolar lavage cellular composition in acute asthma and acute bronchiolitis. J Pediatr 2000; 137:517-22.
3. Hamid Q, Tulic MK, Liu MC, Moqbel R. Inflammatory cells in asthma: mechanisms and implications for therapy. J Allergy Clin.Immunol. 2003; 111:S5-S12.
4. Bateman ED, Hurd SS, Barnes PJ, Bousquet J, Drazen JM, Fitzgerald M, et al. Global strategy for asthma management and prevention: GINA executive summary. Eur Respir J 2008; 31:143-78.
5. Expert Panel Report 3 (EPR3): Guidelines for the Diagnosis and Management of Asthma NHLBI produced publications 2007; Section 3: Overview and Component 1: Measures of Assessment and Monitoring, (452 K) 39.
6. Cicutto LC, Downey GP. Biological markers in diagnosing, monitoring, and treating asthma: a focus on noninvasive measurements. AACN Clin Issues 2004; 15:97-111.
7. Green RH, Brightling CE, McKenna S, Hargadon B, Parker D, Bradding P, et al. Asthma exacerbations and sputum eosinophil counts: a randomised controlled trial. Lancet 2002; 360:1715-21.
8. Brightling CE, Green RH, Pavord ID. Biomarkers predicting response to corticosteroid therapy in asthma. Treat Respir Med 2005; 4:309-16.
9. Holgate ST. Epithelium dysfunction in asthma. J Allergy Clin Immunol 2007; 120:1233-44; quiz 45-6.
10. Bentley JK, Hershenson MB. Airway smooth muscle growth in asthma: proliferation, hypertrophy, and migration. Proc Am Thorac Soc 2008; 5:89-96.
11. Cockcroft DW, Davis BE. Mechanisms of airway hyperresponsiveness. J Allergy Clin Immunol 2006; 118:551-9.
12. Watkins SM, German JB. Metabolomics and biochemical profiling in drug discovery and development. Curr Opin Mol Ther 2002; 4:224-8.
13. Bollard ME, Stanley EG, Lindon JC, Nicholson JK, Holmes E. NMR-based metabonomic approaches for evaluating physiological influences on biofluid composition. NMR Biomed. 2005; 18:143-62.
14. Messana I, Forni F, Ferrari F, Rossi C, Giardina B, Zuppi C. Proton nuclear magnetic resonance spectral profiles of urine in type II diabetic patients. J Clin Chem 1998; 44:1529-34.
15. Umpierrez GE, DiGirolamo M, Tuvlin JA, Isaacs SD, Bhoola SM, Kokko JP. Rapid diagnosis of alcoholic ketoacidosis by proton NMR. Intensive Care Med 2001; 27:785 - 6.
16. Canning BJ. Modeling asthma and COPD in animals: a pointless exercise? Curr. Opin. Pharmacol. 2003; 3:244 - 50.
17. Adamko DJ, Yost BL, Gleich GJ, Fryer AD, Jacoby DB. Ovalbumin sensitization changes the inflammatory response to subsequent parainfluenza infection. Eosinophils mediate airway hyperresponsiveness, M2 muscarinic receptor dysfunction, and antiviral effects. J.Exp Med. 1999; 190:1465-78.
18. Costello RW, Evans CM, Yost BL, Belmonte KE, Gleich GJ, Jacoby DB, et al. Antigen-induced hyperreactivity to histamine: role of the vagus nerves and eosinophils. Am.J.Physiol 1999; 276:L709-L14.
19. Evans CM, Jacoby DB, Fryer AD. Effects of dexamethasone on antigen-induced airway eosinophilia and M(2) receptor dysfunction. Am.J.Respir.Crit Care Med. 2001; 163:1484-92.
20. Adamko DJ, Fryer AD, Bochner BS, Jacoby DB. CD8+ T-lymphocytes in viral hyperreactivity and M2 muscarinic receptor dysfunction. Am.J.Respir.Crit Care Med. 2003; 167:550-6.
21. Becker A, Berube D, Chad Z, Dolovich M, Ducharme F, D'Urzo T, et al. Canadian Pediatric Asthma Consensus guidelines, 2003 (updated to December 2004): introduction. Cmaj 2005; 173:S12-4.
22. Kumar A, Ernst RR, Wuthrich K. A two-dimensional nuclear Overhauser enhancement (2D NOE) experiment for the elucidation of complete proton-proton cross-relaxation networks in biological macromolecules. Biochem Biophys Res Commun 1980; 95:1-6.
23. Saude EJ, Slupsky CM, Sykes BD. Optimization of NMR analysis of biological fluids for quantitative accuracy.. Metabolomics 2006; 2:113-23.
24. Saude EJ, Adamko D, Rowe BH, Marrie T, Sykes BD. Variation of metabolites in normal human urine. Metabolomics 2007; 3:439-51.
25. Saude EJ, Sykes BD. Urine stability for metabolomic studies: effects of preparation and storage. Metabolomics 2007; 3:19-27.
26. Slupsky CM, Rankin KN, Wagner J, Fu H, Chang D, Weljie AM, et al. Investigations of the effects of gender, diurnal variation, and age in human urinary metabolomic profiles. Anal Chem 2007; 79:6995-7004.
27. Somorjai RL, Dolenko B, Demko A, Mandelzweig M, Nikulin AE, Baumgartner R, et al. Mapping High-Dimensional Data onto a Relative Distance Plane - An Exact Method for Visualizing, Characterizing and Classifying High-Dimensional Patterns. J Biomedical Informatics 2004; 37:366-79.
28. Somorjai R, Alexander M, Baumgartner R, Booth S, Bowman C, Demko A, et al. A Data-Driven, Flexible Machine Learning Strategy for the Classification of Biomedical Data. Artificial Intelligence Methods and Tools for Systems Biology. In: Dubitzky WaA, F, editor. Computational Biology Series: Springer; 2004. p. 67-85.
29. Nikulin AE, B . Dolenko, T. Bezabeh, R.L. Somorjai. Near-optimal region selection for feature space reduction: novel preprocessing methods for classifying MR spectra. NMR Biomed 1998; 11:209-16.
30. Green R, Brightling C, McKenna S, Hargadon B, Parker D, Bradding P, et al. Asthma exacerbations and sputum eosinophil counts: a randomised controlled trial. Lancet 2002; 360:1715 -21.
31. Petsky H, Kynaston J, Turner C, Li A, Cates C, Lasserson T, et al. Tailored interventions based on sputum eosinophils versus clinical symptoms for asthma in children and adults. Cochrane.Database.Syst.Rev. 2007:CD005603.
32. Brightling CE, Green RH, Pavord ID. Biomarkers predicting response to corticosteroid therapy in asthma. Treat Respir Med. 2005; 4:309-16.
33. Saude EJ, Lacy P, Musat-Marcu S, Mayes DC, Bagu J, Man SF, et al. NMR analysis of neutrophil activation in sputum samples from patients with cystic fibrosis. Magn Reson.Med. 2004; 52:807.
34. Denburg JA, Sehmi R, Saito H, Pil-Seob J, Inman MD, O'Byrne PM. Systemic aspects of allergic disease: bone marrow responses. J.Allergy Clin.Immunol 2000; 106:S242-S6.
35. Kumps A, Duez P, Mardens Y. Metabolic, nutritional, iatrogenic, and artifactual sources of urinary organic acids: a comprehensive table. Clin Chem 2002; 48:708-17.
36. Xia X, Lemieux ME, Li W, Carroll JS, Brown M, Liu XS, et al. Integrative analysis of HIF binding and transactivation reveals its role in maintaining histone methylation homeostasis. Proc Natl Acad Sci U S A 2009; 106:4260-5.
37. Semenza GL. Targeting HIF-1 for cancer therapy. Nat Rev Cancer 2003; 3:721-32.
38. Aragones J, Fraisl P, Baes M, Carmeliet P. Oxygen sensors at the crossroad of metabolism. Cell Metab 2009; 9:11-22.
39. Shockcor JP, Holmes E. Metabonomic Applications in Toxicity Screening and Disease Diagnosis. Curr. Top. Med. Chem. 2002; 2:35-51.
40. Azmi J, Connelly J, Holmes E, Nicholson JK, Shore RF, Griffin JL. Characterization of the biochemical effects of 1-nitronaphthalene in rats using global metabolic profiling by NMR spectroscopy and pattern recognition. Biomarkers 2005; 10:401-16.
41. Nelson DL, Lehninger AL, Cox MM. Lehninger principles of biochemistry. 5th ed. New York: W.H. Freeman; 2008.
42. Leipnitz G, Seminotti B, Haubrich J, Dalcin MB, Dalcin KB, Solano A, et al. Evidence that 3-hydroxy-3-methylglutaric acid promotes lipid and protein oxidative damage and reduces the nonenzymatic antioxidant defenses in rat cerebral cortex. J Neurosci Res 2008; 86:683-93.
43. Saude EJ, Obiefuna IP, Somorjai RL, Ajamian F, Skappak C, Ahmad T, et al. Metabolomic biomarkers in a model of asthma exacerbation: urine nuclear magnetic resonance. Am J Respir Crit Care Med 2009; 179:25-34.
44. Pauly DF, Pepine CJ. The role of carnitine in myocardial dysfunction. Am J Kidney Dis 2003; 41:S35-43.
45. Oka T, Itoi T, Terada N, Nakanishi H, Taguchi R, Hamaoka K. Change in the membranous lipid composition accelerates lipid peroxidation in young rat hearts subjected to 2 weeks of hypoxia followed by hyperoxia. Circ J 2008; 72:1359-66.
46. Brown RA, Spina D, Page CP. Adenosine receptors and asthma. British Journal of Pharmacology 2008; 153:S446-S56.
47. Benedict JD, Kalinsky HJ, Scarrone LA, Wertbeim AR, Stetten D, Jr. The origin of urinary creatine in progressive muscular dystrophy. J Clin Invest 1955; 34:141-5.
48. Fitch C, Sinton D. A Study of Creatine Metabolism in Diseases Causing Muscle Wasting. J Clin Invest 1964; 43:444 - 52.
49. Baker PR, Cramer SD, Kennedy M, Assimos DG, Holmes RP. Glycolate and glyoxylate metabolism in HepG2 cells. Am J Physiol Cell Physiol 2004; 287:C1359-65.
50. Bekier E, Wyczolkowska J, Szyc H, Maslinski C. The inhibitory effect of nicotinamide on asthma-like symptoms and eosinophilia in guinea pigs, anaphylactic mast cell degranulation in mice, and histamine release from rat isolated peritoneal mast cells by compound 48-80. Int Arch Allergy Appl Immunol 1974; 47:737-48.
51. Barnes PJ. Neural control of human airways in health and disease. Am Rev Respir Dis 1986; 134:1289-314.
52. Okuyama K, Ohwada K, Sakurada S, Sato N, Sora I, Tamura G, et al. The distinctive effects of acute and chronic psychological stress on airway inflammation in a murine model of allergic asthma. Allergol Int 2007; 56:29-35.
53. Kang DH, Fox C. Neuroendocrine and leukocyte responses and pulmonary function to acute stressors. Ann Behav Med 2000; 22:276-85.
54. Ilias I, Pacak K. Diagnosis and management of tumors of the adrenal medulla. Horm Metab Res 2005; 37:717-21.
55. Wu W, Samoszuk MK, Comhair SA, Thomassen MJ, Farver CF, Dweik RA, et al. Eosinophils generate brominating oxidants in allergen-induced asthma. J.Clin.Invest 2000; 105:1455-63.
56. Nishiwaki F, Kuroda K, Inoue Y, Endo G. Determination of histamine, 1-methylhistamine and N-methylhistamine by capillary electrophoresis with micelles. Biomed Chromatogr 2000; 14:184-7.
57. Takei S, Shimago A, Iwashita M, Kumamoto T, Kamuro K, Miyata K. Urinary N-methylhistamine in asthmatic children receiving azelastine hydrochloride. Ann Allergy Asthma Immunol 1997; 78:492-6.
58. Stephan V, Zimmermann A, Kuhr J, Urbanek R. Determination of N-methylhistamine in urine as an indicator of histamine release in immediate allergic reactions. J Allergy Clin Immunol 1990; 86:862-8.
59. Tan P, Bharath A. Manipulation of indoleamine 2,3 dioxygenase; a novel therapeutic target for treatment of diseases. Expert Opin Ther Targets 2009.
60. Odemuyiwa SO, Ghahary A, Li Y, Puttagunta 1, Lee JE, Musat-Marcu S, et al. Cutting edge: human eosinophils regulate T cell subset selection through indoleamine 2,3-dioxygenase. J.Immunol. 2004; 173:5909-13.
61. Reynolds RD, Natta CL. Depressed plasma pyridoxal phosphate concentrations in adult asthmatics. Am J Clin Nutr 1985; 41:684-8.
62. Delport R, Ubbink JB, Serfontein WJ, Becker PJ, Walters L. Vitamin B6 nutritional status in asthma: the effect of theophylline therapy on plasma pyridoxal-5'-phosphate and pyridoxal levels. Int J Vitam Nutr Res 1988; 58:67-72.
63. Ullmann F. Ullmann's encyclopedia of industrial chemistry. [New York]: Wiley, 2000.
64. Um SY, Chung MW, Kim KB, Kim SH, Oh JS, Oh HY, et al. Pattern recognition analysis for the prediction of adverse effects by nonsteroidal anti-inflammatory drugs using 1H NMR-based metabolomics in rats. Anal Chem 2009; 81:4734-41.
65. Messana I, Forni F, Ferrari F, Rossi C, Giardina B, Zuppi C. Proton nuclear magnetic resonance spectral profiles of urine in type II diabetic patients. Clin Chem 1998; 44:1529-34.
66. Holmes E, Bonner FW, Sweatman BC, Lindon JC, Beddell CR, Rahr E, et al. Nuclear magnetic resonance spectroscopy and pattern recognition analysis of the biochemical processes associated with the progression of and recovery from nephrotoxic lesions in the rat induced by mercury(II) chloride and 2-bromoethanamine. Mol Pharmacol 1992; 42:922-30.
67. Lu Y, Zhao H, Zhang C, Lai Q, Wu X, Xing XH. Expression of NAD(+)-dependent formate dehydrogenase in Enterobacter aerogenes and its involvement in anaerobic metabolism and H(2) production. Biotechnol Lett 2009.
68. U.S. patents. [S.1.: s.n., 2009.
69. Freund-Michel V, Frossard N. The nerve growth factor and its receptors in airway inflammatory diseases. Pharmacol Ther 2008; 117:52-76.
70. Blanchet MR, Langlois A, Israel-Assayag E, Beaulieu MJ, Ferland C, Laviolette M, et al. Modulation of eosinophil activation in vitro by a nicotinic receptor agonist. J Leukoc Biol 2007; 81:1245-51.
71. Moldave K, Meister A. Synthesis of phenylacetylglutamine by human tissue. J Biol Chem 1957; 229:463-76.
72. Delaney J, Neville WA, Swain A, Miles A, Leonard MS, Waterfield CJ. Phenylacetylglycine, a putative biomarker of phospholipidosis: its origins and relevance to phospholipid accumulation using amiodarone treated rats as a model. Biomarkers 2004; 9:271-90.
73. Nicholls AW, Mortishire-Smith RJ, Nicholson JK. NMR Spectroscopic-Based Metabonomic Studies of Urinary Metabolite Variation in Acclimatizing Germ-Free Rats. Chem. Res. Toxicol. 2003; 16:1395-404.
74. Khiabani KT, Stephenson LL, Gabriel A, Nataraj C, Wang WZ, Zamboni WA. A quantitative method for determining polarization of neutrophil adhesion molecules associated with ischemia reperfusion. Plast.Reconstr.Surg. 2004; 114:1846-50.
75. Del Giudice MM, Rocco A, Capristo C. Probiotics in the atopic march: highlights and new insights. Dig Liver Dis. 2006; 38:S288-90.
76. Braunwald E. Biomarkers in heart failure. N Engl J Med 2008; 358:2148-59.
77. Petsky HL, Cates CJ, Li AM, Kynaston JA, Turner C, Chang AB. Tailored interventions based on exhaled nitric oxide versus clinical symptoms for asthma in children and adults. Cochrane Database Syst Rev 2008:CD006340.
78. Carraro S, Rezzi S, Reniero F, Heberger K, Giordano G, Zanconato S, et al. Metabolomics Applied to Exhaled Breath Condensate in Childhood Asthma. Am. J. Respir. Crit. Care Med. 2007:200606-769OC.
79. Rabinovitch N. Urinary leukotriene E4. Immunol Allergy Clin North Am 2007; 27:651-64; vii.
80. Ko FW, Leung TF, Hui DS. Are exhaled breath condensates useful in monitoring asthma? Curr Allergy Asthma Rep 2007; 7:65-71.

## Claims

1. A method of diagnosing an asthma disease state in a subject comprising:
(a) determining the concentration of each of 1-methylhistamine, 1-methylnicotinamide, 2-methylglutarate, 2-oxoglutarate, 3-OH-3-methyl glutarate, 3-methyladipate, 4-aminohippurate, acetone, adenine, alanine, creatine, dimethylamine, formate, fumarate, glucose, glycolate, imidazole, lactate, methylamine, O-acetylcarnitine, oxalacetate, phenylacetylglycine, phenylalanine, tryptophan, tyrosine, cis-aconitate, myo-inositol, and trans-aconitate from nuclear magnetic resonance spectroscopic data
obtained from a biofluid sample from the subject;
(b) determining a subject profile for the biofluid sample based on a statistical analysis of said concentration values, wherein the statistical analysis is a multivariate statistical analysis selected from the group consisting of principal component analysis, discriminant analysis, principal component analysis with discriminant analysis, partial least squares, partial least squares with discriminant analysis, canonical correlation, kernel principal component analysis, non-linear principal component analysis, factor analysis, multidimensional scaling and cluster analysis;
(c) comparing the subject profile to a predetermined profile, for differentiating between a first disease state and a second disease state, wherein the predetermined profile is based on statistical analysis of the concentration values of each of 1-methylhistamine, 1-methylnicotinamide, 2-methylglutarate, 2-oxoglutarate, 3-OH-3-methyl glutarate, 3-methyladipate, 4-aminohippurate, acetone, adenine, alanine, creatine, dimethyl amine, formate, fumarate, glucose, glycolate, imidazole, lactate, methylamine, O-acetylcarnitine, oxalacetate, phenylacetylglycine, phenylalanine, tryptophan, tyrosine, cis-aconitate, myo-inositol, and trans-aconitate in each of a plurality of biological samples obtained from chronic stable asthma patients and exacerbated asthma patients;
wherein the first disease state is chronic stable asthma, and the second disease state is exacerbated asthma to provide an asthma diagnosis.

2. The method of claim 1, wherein the nuclear magnetic resonance data comprise xy-trace data.

3. The method of any one of claims 1 to 2, wherein the biofluid sample is a urine sample.

4. The method of any one of claims 1-3, wherein the predetermined profile is a score plot determined using multivariate statistical analysis.

## Patentansprüche

1. Verfahren zur Bestimmung des Stadiums einer Asthma-Erkrankung in einem Individuum, bei dem man :
(a) jeweils die Konzentration von 1-Methylhistamin, 1-Methylnicotinamid, 2-Methylglutarat, 2-Oxoglutarat, 3-OH-3-Methylglutarat, 3-Methyladipat, 4-Aminohippurat, Aceton, Adenin, Alanin, Kreatin, Dimethylamin, Formiat, Fumarat, Glucose, Glycolat, Imidazol, Lactat, Methylamin, O-Acetylcarnitin, Oxalacetat, Phenylacetylglycin, Phenylalanin, Tryptophan, Tyrosin, cis-Aconitat, myo-Inositol und Transaconitat ausgehend von Daten, die mittels einer magnetischen Kernresonanz-Spektroskopie von einer Probe einer biologischen Flüssigkeit eines Individu-ums erhalten wurden, bestimmt;
(b) ein Profil der Probe der biologischen Flüssigkeit für das Individuum basierend auf einer statistischen Analyse der Konzentrationswerte bestimmt, wobei die statistische Analyse eine multivariate statistische Analyse ist, die ausgewählt ist aus der Gruppe bestehend aus Hauptkomponentenanalyse (principal component analysis), Diskriminanzanalyse, Hauptkomponentenanalyse mit Diskriminanzanalyse, Partial Least Squares, Partial Least Squares mit Diskriminanzanalyse, kanonische Korrelation, Kernel-Hauptkomponentenanalyse, nicht-lineare Hauptkomponentenanalyse, Faktor-Analyse, multidimensionale Skalierung und Cluster-Analyse;
(c) das Profil des Individuums mit einem vorbestimmten Profil vergleicht, um zwischen einem ersten Zustand der Erkrankung und einem zweiten Zustand der Erkrankung zu unterscheiden, wobei das vorbestimmte Profil auf einer statistischen Analyse der Konzentrationswerte von jeweils 1-Methylhistamin, 1-Methylnicotinamid, 2-Methylglutarat, 2-Oxoglutarat, 3-OH-3-Methylglutarat, 3-Methyladipat, 4-Aminohippurat, Aceton, Adenin, Alanin, Kreatin, Dimethylamin, Formiat, Fumarat, Glucose, Glycolat, Imidazol, Lactat, Methylamin, O-Acetylcarnitin, Oxalacetat, Phenylacetylglycin, Phenylalanin, Tryptophan, Tyrosin, cis-Aconitat, myo-Inositol und Transaconitat in jeder von einer Mehrzahl von biologischen Proben basiert, die von chronisch stabilen Asthma-Patienten und von Patienten mit schwerem Asthma erhalten wurden;
wobei der erste Zustand der Erkrankung stabiles chronisches Asthma ist und der zweite Zustand der Erkrankung schweres Asthma ist, um eine Asthmadiagnose bereitzustellen.

2. Verfahren nach Anspruch 1, wobei die magnetischen Kernresonanz-Daten xy-trace-Daten umfassen.

3. Verfahren nach einem der Ansprüche 1 bis 2, wobei die Probe einer biologischen Flüssigkeit eine Urinprobe ist.

4. Verfahren nach einem der Ansprüche 1 bis 3, wobei das vorbestimmte Profil ein Score-Plot ist, der mittels multivariater statistischer Analyse bestimmt wurde.

## Revendications

1. Procédé de diagnostic d'un état pathologique d'asthme chez un sujet comprenant :
(a) la détermination de la concentration de chacun de la 1-méthylhistamine, du 1-méthylnicotinamide, du 2-méthylglutarate, du 2-oxoglutarate, du 3-OH-3-méthyl-glutarate, du 3-méthyladipate, du 4-aminohippurate, de l'acétone, de l'adénine, de l'alanine, de la créatine, de la diméthylamine, du formiate, du fumarate, du glucose, du glycolate, de l'imidazole, du lactate, de la méthylamine, de l'O-acétylcarnitine, de l'oxaloacétate, de la phénylacétylglycine, de la phénylalanine, du tryptophane, de la tyrosine, du cis-aconitate, du myo-inositol, et du trans-aconitate à partir de données spectroscopiques de résonance magnétique nucléaire obtenues à partir d'un échantillon de fluide biologique provenant du sujet ;
(b) la détermination du profil du sujet pour l'échantillon de fluide biologique en se basant sur une analyse statistique desdites valeurs des concentrations où l'analyse statistique est une analyse statistique multivariée choisie dans le groupe constitué par une analyse en composantes principales, une analyse discriminante, une analyse en composantes principales avec analyse discriminante, les moindres carrés partiels, les moindres carrés partiels avec une analyse discriminante, une corrélation canonique, une analyse en composantes principales à noyau, une analyse en composantes principales non linéaire, une analyse factorielle, un positionnement multidimensionnel et une analyse typologique ;
(c) la comparaison du profil du sujet avec un profil prédéterminé, pour faire la différenciation entre un premier état pathologique et un second état pathologique, où le profil prédéterminé est basé sur une analyse statistique des valeurs des concentrations de chacun de la 1-méthylhistamine, du 1-méthyl-nicotinamide, du 2-méthylglutarate, du 2-oxoglutarate, du 3-OH-3-méthylglutarate, du 3-méthyladipate, du 4-aminohippurate, de l'acétone, de l'adénine, de l'alanine, de la créatine, de la diméthylamine, du formiate, du fumarate, du glucose, du glycolate, de l'imidazole, du lactate, de la méthylamine, de l'O-acétylcarnitine, de l'oxaloacétate, de la phénylacétylglycine, de la phénylalanine, du tryptophane, de la tyrosine, du cis-aconitate, du myo-inositol, et du trans-aconitate dans chacun d'une pluralité d'échantillons biologiques obtenus à partir de patients souffrant d'un asthme stable chronique et de patients souffrant d'un asthme exacerbé ;
dans lequel le premier état pathologique est un asthme stable chronique, et le second état pathologique est un asthme exacerbé pour fournir un diagnostic d'asthme.

2. Procédé selon la revendication 1, dans lequel les données de résonance magnétique nucléaire comprennent des données de tracé xy.

3. Procédé selon l'une quelconque des revendications 1 et 2, dans lequel l'échantillon de fluide biologique est un échantillon d'urine.

4. Procédé selon l'une quelconque des revendications 1 à 3, dans lequel le profil prédéterminé est un tracé de scores déterminé en utilisant une analyse statistique multivariée.
